# EUROPEAN PATENT APPLICATION

(11) **EP 3 945 135 A1**
(43) Date of publication of application: **02.02.2022**
(21) Application number: 20315363.0
(22) Date of filing: 27.07.2020
(51) Int. Cl.: C12Q 1/6886

(54) **BIOMARKERS FOR DIAGNOSING AND MONITORING LUNG CANCER**

(71) Applicant: Les Laboratoires Servier, 92284 Suresnes (FR); Université de Paris, 75006 Paris (FR); Centre National de la Recherche Scientifique - CNRS, 75794 Paris Cedex 16 (FR); Institut National de la Santé et de la Recherche Médicale (INSERM), 75654 Paris Cedex 13 (FR); ASSISTANCE PUBLIQUE, HOPITAUX DE PARIS, 75004 Paris (FR); SORBONNE UNIVERSITE, 75006 Paris (FR)
(72) Inventor: Lockhart, Brian, 78810 Feucherolles (FR); Guigal-Stephan, Nolwen, 75017 Paris (FR); Xuereb, Laura, 75017 Paris (FR); Taly, Valérie, 92340 Bourg LA Reine (FR); Laurent-Puig, Pierre, 92190 Meudon (FR); Wang-Renault, Shufang, 92340 Bourg LA Reine (FR); Blons, Hélène, 92130 Issy LES Moulineaux (FR)
(74) Representative: Bestel, Delphine

(57) **Abstract**

The present invention relates to the field of oncology, particularly the early diagnostic of lung cancer, the monitoring of lung cancer patients and the prediction of the clinical outcome of patients. The invention relates to a method for diagnosing lung cancer or diagnosing relapse of lung cancer in patients by determination of the level of methylation of gene(s) and the kit to implement said method.

## Description

### FIELD OF THE INVENTION

The present invention relates to the field of oncology, particularly the early diagnostic of lung cancer and the longitudinal monitoring of lung cancer patients. The invention can be used for monitoring the evolution of lung cancer in a subject diagnosed for lung cancer as well as determining or adapting a suitable therapeutic regimen for a said subject. The present invention relates to a list of markers comprising primers and/or probes to detect, determine, and identify hyper-methylated genes in blood plasma samples.

### BACKGROUND OF THE INVENTION

Lung cancer is the most common cause of global cancer-related mortality, leading to over a million deaths each year (Bray, F. et al. Global cancer statistics 2018: GLOBOCAN estimates of incidence and mortality worldwide for 36 cancers in 185 countries. CA Cancer J Clin. 2018 Nov 68(6):394-424). Lung cancer has a poor prognosis: over half of people diagnosed with lung cancer die within one year of diagnosis and the 5-year survival is less than 18% (15% for men, 21 % for women) (Cecilia Zappa and Shaker A. Mousa Non-small cell lung cancer: current treatment and future advances. Transl Lung Cancer Res. 2016 Jun; 5(3): 288-300).

There are two major types of lung cancer, non-small cell lung cancer (NSCLC) and small cell lung cancer (SCLC). Non-small-cell lung cancer represents the most common type, accounting for more than 80% of cases, and can be squamous cell carcinoma, adenocarcinoma or large-cell carcinoma. Small-cell lung cancer is a less common lung cancer type with a more aggressive progression than non-small cell lung cancer. The type of lung cancer determines which treatments are recommended.

Staging lung cancer is based on whether the cancer is local or has spread from the lungs to the lymph nodes or other organs. Because the lungs are large, tumors can grow in them for a long time before detection. Even when symptoms -such as coughing and fatigue- do occur, they are often confounded with other causes. For this reason, early-stage lung cancer is difficult to detect.

While smoking is linked to more than 80% of all lung cancer cases, many people that have never smoked or been exposed to passive smoke develop lung cancer. Of those newly diagnosed with lung cancer, it is estimated that less than 40% are current smokers, more than 45% are former smokers, and 10% to 15% have never smoked.

Depending on the staging and type of lung cancer, patients are eligible for certain treatments ranging from surgery to radiation and/or chemotherapy as well as targeted therapy. With the advancement of molecular genetics and biomarker testing, specific mutations and genomic aberrations (e.g. gene fusions) have been identified that enable improved target treatment for individual patients.

Radical surgery is the standard of care for stage I non-small cell lung cancer (NSCLC) patients. Stage II and IIIA adjuvant cisplatin-based chemotherapy remains the gold standard for completely resected NSCLC tumors. Additionally, radiotherapy should be offered in patients with N2 lymph nodes. In advanced stage IIIB/IV or inoperable NSCLC patients, a multidisciplinary treatment is scheduled consisting of 4 cycles of cisplatin-based chemotherapy plus a third-generation cytotoxic agent or a cytostatic (anti-EGFR, anti-VEGFR) drug.

Recently, molecularly targeted therapies have dramatically improved treatment for patients whose tumors harbor somatically activated oncogenes such as mutant EGFR (Paez, J. G. et al. EGFR mutations in lung cancer: correlation with clinical response to gefitinib therapy. Science 304, 1497-1500 (2004)) or translocated ALK, RET, or ROS1 (Kwak, E. L. et al. Anaplastic lymphoma kinase inhibition in non-small-cell lung cancer. N. Engl. J. Med. 363, 1693-1703 (2010)). Mutant BRAF and ERBB2 (Stephens, P. et al. Lung cancer: intragenic ERBB2 kinase mutations in tumors. Nature 431, 525-526 (2004)) are also investigational targets. However, most lung adenocarcinomas either lack an identifiable driver oncogene, or harbor mutations in KRAS and are therefore still treated with conventional chemotherapy. Tumor suppressor gene abnormalities, such as those in TP53 (Takahashi, T. et al. p53: a frequent target for genetic abnormalities in lung cancer. Science 246, 491-494 (1989)), STK11 (Sanchez-Cespedes, M. et al. Inactivation of LKB1/STK11 is a common event in adenocarcinomas of the lung. Cancer Res. 62, 3659-3662 (2002)), CDKN2A8 (Shapiro, G.I. et al. Reciprocal Rb inactivation and p16INK4 expression in primary lung cancers and cell lines. Cancer Res. 55, 505-509 (1995)), KEAP1 (Singh, A. et al. Dysfunctional KEAP1-NRF2 interaction in non-small-cell lung cancer. PLoS Med. 3, e420 (2006)), and SMARCA4 (Medina, P. P. et al. Frequent BRG1/SMARCA4-inactivating mutations in human lung cancer cell lines. Hum. Mutat. 29, 617-622 (2008)) are also common but are not, at the present time, clinically actionable. Finally, lung adenocarcinomas show high rates of somatic mutations and genomic rearrangements, challenging identification of all but the most frequent driver gene alterations because of a large burden of passenger events per tumor genome (Ding, L. et al. Somatic mutations affect key pathways in lung adenocarcinoma. Nature 455, 1069-1075 (2008)).

The TNM (Tumour, Necrosis, Metastases) staging indicates the level of disease progression and the malignant potential of the primary lung cancer (Brundage MD, Davies D, Mackillop WJ. Prognostic factors in non-small cell lung cancer: a decade of progress. Chest 2002;122:1037-57). However, even patients with disease at the same stage exhibit wide variations in their incidence of recurrence after curative resection. Therefore, the current TNM staging system, which is based on clinical and pathological findings, may have reached the limit of its usefulness or at least need complementary tools.

Tumor markers, such as CEA, were proven to be independent factors predicting the risk of recurrence (Kawachi R, et al. Early recurrence after surgical resection in patients with pathological stage I non-small cell lung cancer. Thorac Cardiovasc Surg 2009;57:472-5). Furthermore, the physical examination is very important, because a lower performance status (PS) and the presence of symptoms are unfavorable prognostic factors for the disease free survival (DFS) (Pasini F, et al. Late events and clinical prognostic factors in stage I non-small cell lung cancer. Lung Cancer 2002;37:171-7). The standard uptake value (SUV) in Positron Emission Tomography (PET) has also been reported to be a significant independent factor predicting the DFS (Shiono S, et al. Positron emission tomography/computed tomography and lymphovascular invasion predict recurrence in stage I lung cancers. J Thorac Oncol 2011;6:43-7). An extensive pathological investigation is also important, because the histological differentiation, vessel invasion, lymphatic permeation and pleural invasion have all been reported to be poor prognostic factors for the DFS (Maeda R, et al. Risk factors for tumor recurrence in patients with early-stage (stage I and II) non-small cell lung cancer: patient selection criteria for adjuvant chemotherapy according to the seventh edition TNM classification. Chest 2011; 140:1494-502). Furthermore, complete mediastinal lymph node (MLN) dissection is associated with improved survival in comparison to random lymph node sampling for the patients with stage I NSCLC (Gajra A, et al. Effect of number of lymph nodes sampled on outcome in patients with stage I non-small-cell lung cancer. J Clin Oncol 2003;21:1029-34), although there have been some negative reports.

In this context, there is still a need to identify sensitive and specific biomarkers of lung cancer that can be used on body fluid, as a minimally invasive means for diagnosing lung cancer and its recurrence as well as monitoring the evolution of cancer during the treatment and adjusting a therapeutic regimen of the patient according to said monitoring.

Analysis of circulating tumor DNA (ctDNA) in plasma has recently emerged as a promising approach allowing systematic and real-time monitoring of the whole genomic alterations of cancer patients (Cagle, P.T. et al., Lung cancer biomarkers: present status and future developments. Arch Pathol Lab Med, 2013. 137(9): p. 1191-8.).

The ability to study non-hematologic cancers through non-invasive blood sampling is one of the most exciting and rapidly advancing fields in cancer theranostics. Invasive and repetitive tissue biopsies to monitor primary and metastatic tumor evolution are problematic because of the inherent risk to the patient and evident cost issues, and their limitation to inform on the global heterogeneity of the cancer. The demonstration that plasma from cancer patients contains cell-free DNA originating from the tumors that carries information on the tumor's somatic genetic abnormalities has the potential to inform on tumor genetics, tumor burden, tumor heterogeneity, and mechanisms of response/progression and drug resistance (Heitzer, E. et al., Current and future perspectives of liquid biopsies in genomics-driven oncology. Nature Reviews Genetics volume 20, pages71-88 (2019)).

The existence of circulating cell-free DNA (ccfDNA), constantly released into the bloodstream in healthy individuals, has been known for several decades. Indeed, fragments of DNA are shed into the blood from dying cells during cellular turnover or other forms of cell death (Schwarzenbach, H. et al., Cell-free nucleic acids as biomarkers in cancer patients. Nature Reviews Cancer volume11, pages 426-437 (2011)). This highly fragmented double-stranded DNA is mostly composed of molecules of approximately 150 bp in length corresponding to the length of internucleosomal DNA. In non-pathologic conditions, apoptotic or necrotic cells are cleared, and the levels of ccfDNA are relatively low. More than 90% of healthy individuals have less than 25 ng ccfDNA per mL (Adalsteinsson VA et al. Scalable whole-exome sequencing of cell-free DNA reveals high concordance with metastatic tumors. Nat Commun. 2017 Nov 6;8(1):1324).

As mentioned above, cancer cells also release DNA into the bloodstream via cell death and secretion from primary tumors, metastatic lesions, or Circulating Tumor Cells (CTCs). The fraction of ccfDNA that is tumor-derived in patients with cancer has a variable contribution ranging from <0.1% to >10% of the total circulating DNA molecules. ccfDNA levels in plasma from patients with advanced metastatic cancer are typically several-fold higher than those in healthy individuals. However, these levels can vary widely from 0.0 to 1000ng/mL in cancer patients (Adalsteinsson VA et al. Scalable whole-exome sequencing of cell-free DNA reveals high concordance with metastatic tumors. Nat Commun. 2017 Nov 6;8(1):1324). The variability in levels of ctDNA is not well understood and is thought to be affected by tumor burden, stage, metastasis localization, cellular turnover, accessibility to the circulation, proliferation rate, level of necrosis and factors affecting blood volume.

Over the last 5-10 years, the emergence of new more sensitive technologies and more refined methodologies in genomic analysis of plasma from cancer patients in particular with regard to Next-generation Sequencing such as DNASeq (Whole Genome Sequencing, Whole Exome Sequencing, Targeted sequencing), RNASeq (global and targeted) and PCR-based approaches (ddPCR, droplet-based digital-PCR, multiplexing PCR, etc.) have been observed. Many of these approaches whether global or targeted have permitted the detection of multiple genomic anomalies (mutations, methylation, SCNAs, indels, chromosomal abnormalities, translocations, gene fusions, etc.) in the plasma.
The possibility to detect multiple tumor-derived genetic anomalies in the plasma offers a plethora of clinical opportunities for cancer patient theranostics including, early diagnosis, minimal residual disease (MRD) detection and monitoring relapse/progression and response. Furthermore, liquid biopsies have the potential to empower patient care based on a more dynamic and longitudinal monitoring of tumor evolution (Crowley, E et al , Liquid biopsy: monitoring cancer-genetics in the blood. Nat Rev Clin Oncol. 2013 Aug;10(8):472-84). For example, monitoring tumor specific mutations in ctDNA has permitted longitudinal follow-up of patients in different types of cancers including colorectal, lung, breast or pancreatic cancers (Pecuchet, N., et al., Base-Position Error Rate Analysis of Next-Generation Sequencing Applied to Circulating Tumor DNA in Non-Small Cell Lung Cancer: A Prospective Study. PLoS Med, 2016. 13(12): p. e1002199).

However, to permit efficient patient follow-up, a large panel of different genes and mutations are often required since these alterations can vary considerably due to tumor heterogeneity. In addition, slow turn-around-time and cost-effectiveness issues have also limited a more widespread application of approaches based on large gene panel screening in clinical practice.

DNA methylation (both hypo- and hypermethylation) is one of the fundamental epigenetic modifications that regulates gene expression by altering transcriptional accessibility of gene regulatory regions, in both physiological and diseased states. Indeed, aberrant DNA methylation has been demonstrated to occur in cancer where the epigenetic silencing of tumor-suppressor genes is recognized as an early event in cancer onset and in driving tumorigenesis. DNA methylation in the human genome occurs mostly at the cytosine residues in a CpG dinucleotide at the carbon-5 position, resulting in 5-methylcytosine. CpG dinucleotides are rare in the human genome, about 1%. CpG dinucleotides are often found to be in clusters of more than 200 bases with more than 50% of G+C content and a ratio of CpG frequencies of at least 0.6, which are known as CpG islands. In particular, hypermethylation of CpG dense regions (CpG islands) are frequently located at gene promoters and have been associated with silencing of tumor suppressors and incomplete differentiation, directly linking DNA methylation changes to oncogenic transformation (Qureshi, S.A., M.U. Bashir, and A. Yaqinuddin, Utility of DNA methylation markers for diagnosing cancer. Int J Surg, 2010. 8(3): p. 194-8). Since the aberrant DNA methylation often occurs in the early stage of carcinogenesis and is stable, it is considered as an attractive potential marker for the early detection of cancer and also to longitudinally monitor disease burden. Moreover, with the objective of developing biomarkers for cancer screening, monitoring and early detection, the increased methylation and hypermethylation provide a positive readout with clear target regions for sensitive and specific assay development.
Most notably, Taly et al. (Garrigou, S., et al., A Study of Hypermethylated Circulating Tumor DNA as a Universal Colorectal Cancer Biomarker. Clin Chem, 2016. 62(8): p. 1129-39) have shown that monitoring of ctDNA by droplet-based digital PCR (ddPCR) by targeting either methylation markers or tumor-specific mutations lead to comparable results.

In contrast to the complexity of using mutation biomarkers, the analysis of a limited number of methylation biomarkers has the potential to allow screening of large number of cancer patients. Indeed, Taly et al. demonstrated that using only two methylation markers allowed monitoring of all the CRC patients studied. In addition, the pertinence of using these markers for ctDNA detection and monitoring in different prospective results in mCRC has been demonstrated (Garlan, F., et al., Early Evaluation of Circulating Tumor DNA as Marker of Therapeutic Efficacy in Metastatic Colorectal Cancer Patients (PLACOL Study). Clinical Cancer Research, 2017. 23(18): p. 5416-5425).

The present disclosure concerns new DNA methylation biomarkers with high sensibility and specificity for the diagnostic and/or follow-up of lung cancer patients. Identification of tumor-specific methylated markers in ctDNA have the potential to improve tumor detection and patient monitoring and to by-pass some of the existing difficulties associated with following mutation or other genomic events. Therefore, the hypermethylation of the MROH6 gene, HOXB4 gene, chr7:129425301, chr20:1784267 OPLAH gene, CRCT1 gene, KCNQ4 gene, GP5 gene and NPR3 gene referred hereafter to as "biomarker of the invention" in ctDNA have important implications for lung cancer detection and patient management through liquid biopsy.

Advantages of this approach could indeed allow reduction in the quantity of tests to perform, monitoring of tumor DNA dynamics without the need to develop individualized assays for each cancer patient as well as prevent the need to characterize the primitive tumor.

### DETAILED DESCRIPTION

The present disclosure concerns a method for diagnosing lung cancer or diagnosing relapse of lung cancer in a biological sample of a patient by determination of the level of methylation of at least one gene selected in the group consisting of : MROH6 gene, HOXB4 gene, chr7:129425301 gene, chr20:1784267 gene, OPLAH gene, CRCT1 gene, KCNQ4 gene, GP5 gene and NPR3 gene.

As used herein the terms "patient", "subject" or "individual" are used interchangeably and refer to a mammal, preferably a human. The patient may be healthy without any symptoms of lung cancer or thought to develop or is suspected of suffering cancer especially lung cancer. Said patient presents for example at least one of the following symptoms: persistent cough, chest pain, hoarseness, loss of appetite, loss of weight, coughing up blood, shortness of breath, bronchitis or pneumonia that keep recurring. Said patient may also have suffered from a lung cancer in the past, has been treated and is monitored for potential disease recurrence. Said patient may also seem to be healthy but is predisposed to develop lung cancer for genetic predisposition or for family history.

As used herein, the expression "biological sample" refers to solid tissues such as lung biopsy or to fluids, body effluents and excretions such as phlegm, sputum, stools, blood, serum, plasma, urine, feces. Preferably, the biological sample is a blood or plasma sample.

The inventors have shown that hypermethylation of these genes is used as a sensitive and specific biomarker of lung cancer in patients suffering thereof, even at early stage. The MROH6 gene, HOXB4 gene, chr7:129425301, chr20:1784267 OPLAH gene, CRCT1 gene, KCNQ4 gene, GP5 gene and NPR3 gene, are hereafter referred to as "biomarker of the invention" (Table 1).

**Table 1: nucleotide sequence or biomarkers of the invention**

| Region of interest | Nucleotide sequence |
|---|---|
| MROH6 chr8:144650594-144650916 | CCGTCACACCTGCCACTGAGGTCCTTGGGATGGGT GAGTCCCTGACCTGTAATTGTCGGAGGGGAGGCAC GGTGGGAGTGGTGAGTGTTGGATGGCATAGGGGTG GGATGGTGTCGGGGGCTGCTGACCTCAGCAGCGTC CCTGCTGGGGTCATGCAGGCGCAGCAGCAGCGGCA CGAGACTCTGCAGCACCAGCTTCCGCAGGGGGCCG CGGAGCCCCAGCCGGAGCCCGCCCCGGCCCCGGC GCACCAGAGTCCCAAGGAGCCCGACGGCCGAGGC |
| | GCGGATTGAGTCCCGTGTCTGCGTGGGAGGGCGCA GTCAGGGCAG (SEQ ID NO:1) |
| HOXB4 chr17:46656012-46656181 | CGCTCCCTCCCGCCGAGGGGAGGAGACTGGGGCCT TTGGAAGGTACAGAATGCTGGGCAGAGGTGAGGCA GAACAGGAGGGCTACACCGGGAGCCCCAACTTCTT AAGGGATCTAGGGTTTGCGCTCTGCCTAGCGCCCCA ACAGGCCCTCCAGTCGCAACCTGCGCGCG (SEQ ID NO:2) |
| chr7:129425301 chr7:129425301-129426014 | TCGTCTGTAAATTGGGAGCTTACCCAGGTCGGAGGA CTGTTGGAATTGGAAATATTCGAATAAGGAAGTGTTT TTGCAAGTGCTTTGTAAGCAGCAAAGCGCTTCTTCA GGGGTCAATTTTTTTTTAGCTCTGCAGTCACCACCCA AATTCGGAAGATCGTTGTGCCTTTCTTGGATGAGAAT GCCCGGCTCCAGCCCGGGGATGGACGGTCTTTTC CTTGCTAGGAGTCCGCTGTGGAGGGGGCGATGCGC TCTGTCCAGGACCTAGGAGTCCCTTTCTCCCTCCAC CCTCGGCTCACAGCGGGCAGCTCCCCCGACGCGTA CCGCCTCTTTGCCCCCAGCCTGAGGTGCGCTCTTCC CCAGGACGCCTCCTCTGTCCTCTGCACGCCGCCGG CCCTGCTGGGGTTGCTTTCTGCCGCGGCGTCTTCG CGAATATCCCCTCTTGCTGTCTGCCTGGGCGTCCCT CCGGGTTACCGCACACCGCCTCCGACCCTCCCAGG GCGCGGGAGGAGGGGCAGGGCATCCGCGTTCGTG TCTGGGCCCTGGCGGGGAGTCGCGTGGTCCAACCC CTCGTTTTTAAGAGGAGGGATCTGAAGCCCAGCTAG GCTTCTAATTCCAGACAGAGCCCTACGGAGCCCAGT TCTCTGGAGGCTTTCCTGGGGCTTTTTCACTAGGC CAGGTTGGGTGGGTCTGGAGAGAACTCCCGCAACC G (SEQ ID NO:3) |
| chr20:1784267 chr20:1784266-1784458 | CGGGGCGCCGCGGTAGGGGCACTGGAGCGCACGA TGCACCCCGCCAACGAGTCCTTTCTGCAGACGGGG TTCCTGTTTTCATGCAAATGCCTTTGTTAGCGCACCG GGAACCAGGGGGACGGAACTGCAGCTGACGCGGG CTGCGCGCCGCTTTTCCGCCTTCGCTTGATTCGGCC TCAACGACTTAAACGCG (SEQ ID NO:4) |
| OPLAH chr8:1451 06155-145106382 | CGCCAACAGGATGTGTTCTGTCTCCACACGCCCGGC GGCGGTGGCTATGGGGACCCGGAGGACCCCGCCC CACCGCCGGGGTCGCCCCCGCAAGCACTGGCCTTT CCCGAGCACGGCAGCGTCTATGAGTATCGCCGGGC CCAGGAGGCCGTGTGAGGATCCCGCAATAAAGATG CCTTAAGTCTCCCGGTTCTGGGGACGCAGCTACGG CGCCTAGTTCGTGCTCCG (SEQ ID NO:5) |
| CRCT1 chr19:18811515-18811709 | CGCCTGGGACTGGCTCTGTGGCTGGCCGTCCCTC TGGCTGGGGACACGGGGAGGCCGAGCGTGTCCGC AAAGACACGCGAGCTGTCCACCTTTCTGGGCTGCAG GGGTCTGCAGAACCTCGGAACCTCGGAGGGGCTCT CTGTCTGCTTCAGCGTCTCTTTCTCAAGTGACTGAAA GCAGAGGCTCCCTGCTGC (SEQ ID NO:6) |
| KCNQ4 chr1:41284053-41284166 | CCGCACAGATTCAGCGGACCCTCCCCCTCCCTCCC CAGTCCCCTGCCACATCCCCAGAAGTCCCCGCCTCC GGGTCCGTGCGCGGGGTAGGCTGGCTGTGATCTCG CCGCCCCC (SEQ ID NO:7) |
| GP5 chr3:194118923-194119061 | CGCGAACGCTCCTGTGTGTTGACCACACTCCCACGG TTGCTTTTTCAGACATGCTGAGGGGGACTCTACTGT GCGCGGTGCTCGGGCTTCTGCGCGCCCAGCCCTTC CCCTGTCCGCCAGCTTGCAAGTGTGTCTTCCG (SEQ ID NO:8) |
| NPR3 chr5:32713289-32713917 | GCGACACTCACTTCTCCCAGAGTGTTCTGCAACGCA GTTTCTAATGCGCCCAGAGCTAAGCTTTGCGGGAGT GCCTTTTGAAGAAACGAGCGCTGAACCTTCTTTCCA CCTGTCCCCCAAGTTTTCTCCATGGGTCTTTGGGTG TTGGAATAAAACCCAAACTGGACAAAGAGCTCGAGG CTGAGGATGGTGGCTATGGCTTCGGGGAGCGGGGG GACGCGGACGTGTAATCGCCAGTGTGGCCCATTTT ACGGTAGGGTAGAATCTGAGGGAAGGCGGACTGAG ATGCCGGTATAGCGCCGATCCCTCCTCCCTCTAGTC AGGGATTAGGAGCACTGCGATGAGGGAATCCCGTC TCTGCTCCCCCTTGGCGCTCACGCGCCTGGAATGTG AGTGGTGCAATCCCGGCAAAGCTCCCCGAGACCCC AGTGCCACGCGGATGAGACCGAAGGGGAGCGGAGT TGAGCCAGGCGCCGGGCTAGTCCACAACTTGGCAT GGCCGAGCACCTGGGCGCTGGTCGTAGGGGACAG CGCAACCTGAGGCCGAAGGCCCCAGGAACAGGTGA GAGTGGACAGAGAAGCCATCGATAATGGCGCTGCT GTCACCGCCTTGCCATCTTGGCCACCTC (SEQ ID NO:9) |

The method of the invention requires the detection of the "level of methylation", "methylation level" or the "amount of methylation", depending on the detection technology which is used. These terms can be used interchangeably and refer to the determination of a quantitative measure.

As used herein, "relapse" or "recurrence" means the risk that a cancer that has been treated will recur.

In a preferred embodiment, the present disclosure concerns a diagnostic method or a diagnostic method of relapse of lung cancer by determination of the level of methylation of at least one gene selected in the group consisting of: MROH6 gene, HOXB4 gene, chr7:129425301 gene, chr20:1784267 gene and OPLAH gene in a biological sample of a patient.

In a more preferred embodiment, the present disclosure concerns a diagnostic method or a diagnostic method of relapse of lung cancer by determination, in a biological sample of a patient, of the level of methylation of at least one gene selected in the four genes group: MROH6 gene, HOXB4 gene, chr7:129425301 gene, chr20:1784267 gene.

In an aspect the diagnostic method of lung cancer or the diagnostic method of relapse of lung cancer consists in the determination of the level of methylation in a biological sample of a patient of a combination of at least two genes, three genes, four genes, five genes, six genes, seven genes, eight genes or nine genes selected in the group consisting of : MROH6 gene, HOXB4 gene, chr7:129425301 gene, chr20:1784267 gene, OPLAH gene, CRCT1 gene, KCNQ4 gene, GP5 gene and NPR3 gene.

Preferred combinations of two biomarkers are disclosed in Table 2. The preferred combinations of two biomarkers are colored in grey in Table 2.

**Table 2: combinations of two biomarkers**

| Genes | MROH6 | HOXB4 | chr7 | chr20 | OPLAH | CRCT1 | KCNQ4 | GP5 | NPR3 |
|---|---|---|---|---|---|---|---|---|---|
| MROH6 | | MROH6 | MROH6 | MROH6 | MROH6 | MROH6 | MROH6 | MROH6 | MROH6 |
| | | HOXB4 | chr7 | chr20 | OPLAH | CRCT1 | KCNQ4 | GP5 | NPR3 |
| HOXB4 | HOXB4 | | HOXB4 | HOXB4 | HOXB4 | HOXB4 | HOXB4 | HOXB4 | HOXB4 |
| | MROH6 | | chr7 | chr20 | OPLAH | CRCT1 | KCNQ4 | GP5 | NPR3 |
| chr7 | chr7 | chr7 | | chr7 | chr7 | chr7 | chr7 | chr7 | chr7 |
| | MROH6 | HOXB4 | | chr20 | OPLAH | CRCT1 | KCNQ4 | GP5 | NPR3 |
| chr20 | chr20 | chr20 | chr20 | | chr20 | chr20 | chr20 | chr20 | chr20 |
| | MROH6 | HOXB4 | chr7 | | OPLAH | CRCT1 | KCNQ4 | GP5 | NPR3 |
| OPLAH | OPLAH | OPLAH | OPLAH | OPLAH | | OPLAH | OPLAH | OPLAH | OPLAH |
| | MROH6 | HOXB4 | chr7 | chr20 | | CRCT1 | KCNQ4 | GP5 | NPR3 |
| CRCT1 | CRCT1 | CRCT1 | CRCT1 | CRCT1 | CRCT1 | | CRCT1 | CRCT1 | CRCT1 |
| | MROH6 | HOXB4 | chr7 | chr20 | OPLAH | | KCNQ4 | GP5 | NPR3 |
| KCNQ4 | KCNQ4 | KCNQ4 | KCNQ4 | KCNQ4 | KCNQ4 | KCNQ4 | | KCNQ4 | KCNQ4 |
| | MROH6 | HOXB4 | chr7 | chr20 | OPLAH | CRCT1 | | GP5 | NPR3 |
| GP5 | GP5 | GP5 | GP5 | GP5 | GP5 | GP5 | GP5 | | GP5 |
| | MROH6 | HOXB4 | chr7 | chr20 | OPLAH | CRCT1 | KCNQ4 | | NPR3 |
| NPR3 | NPR3 | NPR3 | NPR3 | NPR3 | NPR3 | NPR3 | NPR3 | NPR3 | |
| | MROH6 | HOXB4 | chr7 | chr20 | OPLAH | CRCT1 | KCNQ4 | GP5 | |

The method of diagnostic according to the disclosure is based on an hypermethylation of at least one biomarker of the present invention. An "hypermethylation" means a methylation value of any of the biomarker of the invention which is significantly higher in the biological sample of the tested subject as compared with the methylation value of the corresponding biomarker measured in a reference sample. A significantly higher amount of at least one biomarker of the invention in the biological sample of a subject as compared with the normal amount of methylation in the reference sample is an indication that the tested patient has a lung cancer or has a high risk to have a lung cancer or to relapse of lung cancer.

A significantly higher amount or level of methylation refers to a methylation amount that is greater than the standard error of the assay employed to assess said level of methylation.

In another embodiment, the method for diagnosing lung cancer or diagnosing relapse of lung cancer in the present disclosure comprises:
a) assessing in a biological sample of a patient the level of methylation of at least one gene selected in the group consisting of: MROH6 gene, HOXB4 gene, OPLAH gene, CRCT1 gene, KCNQ4 gene, GP5 gene, NPR3 gene, chr7:129425301 gene and chr20:1784267 gene; and
b) comparing the level of methylation in (a) with the level of methylation for the same gene(s) in a control sample;
wherein the level of methylation in (a) superior to the level of methylation in (b) is indicative of lung cancer.

In a more preferred embodiment, the step (a) of the method for diagnosing consists in the assessment of the level of methylation of at least one gene selected in the group consisting of 5 genes: MROH6 gene, HOXB4 gene, chr7:129425301 gene, chr20:1784267 gene and OPLAH gene in a biological sample of a patient.

In another preferred embodiment, the step (a) of the method for diagnosing consists in the assessment of the level of methylation of at least one gene selected in the four genes group: MROH6 gene, HOXB4 gene, chr7:129425301 gene, chr20:1784267 gene in a biological sample of a patient.

In an aspect the step (a) of the diagnostic method of lung cancer or the diagnostic method of relapse of lung cancer consists in the assessment of the level of methylation in a biological sample of a patient of a combination of at least two genes, three genes, four genes, five genes, six genes, seven genes, eight genes or nine genes selected in the group consisting of : MROH6 gene, HOXB4 gene, chr7:129425301 gene, chr20:1784267 gene, OPLAH gene, CRCT1 gene, KCNQ4 gene, GP5 gene and NPR3 gene.

The present disclosure concerns a method for diagnosing lung cancer or diagnosing relapse of lung cancer of a patient in a biological sample thereof by determination of the level of methylation. The level of methylation is determined in at least one nucleotide region selected in the group consisting of : the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene, the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene.

In a preferred embodiment, the level of methylation is determined in at least one nucleotide region selected in the group consisting of: the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene and the nucleotide region of SEQ ID NO:5 in the OPLAH gene.
In a more preferred embodiment, the level of methylation is determined in at least one nucleotide region selected in the group consisting of: the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene and the nucleotide region of SEQ ID NO:4 in the chr20:1784267 gene.
In another preferred embodiment, the level of methylation in the present diagnostic method is determined by a combination of at least two biomarkers, three biomarkers, four biomarkers, five biomarkers, six biomarkers, seven biomarkers, eight biomarkers or nine biomarkers selected in the group consisting of: the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene, the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene.

The method for diagnosing lung cancer or diagnosing relapse of lung cancer consists in the determination of the level of methylation of at least one the gene selected in the group consisting of:
a) MROH6 gene by using the primers of sequences SEQ ID NO:10 and SEQ ID NO:11 and/or the probe of sequence SEQ ID NO:28;
b) HOXB4 gene by using the primers of sequences SEQ ID NO:12 and SEQ ID NO: 13 and/or the probe of sequence SEQ ID NO:29;
c) chr7:129425301 gene by using the primers of sequences SEQ ID NO:14 and SEQ ID NO: 15 and/or the probe of sequence SEQ ID NO:30;
d) chr20:1784267 gene by using the primers of sequences SEQ ID NO: 16 and SEQ ID NO:17 and/or the probe of sequence SEQ ID NO:31.
e) OPLAH gene by using the primers of sequences SEQ ID NO:18 and SEQ ID NO: 19 and/or the probe of sequence SEQ ID NO:32;
f) CRCT1 gene by using the primers of sequences SEQ ID NO:20 and SEQ ID NO:21 and/or the probe of sequence SEQ ID NO:33;
g) KCNQ4 gene by using the primers of sequences SEQ ID NO:22 and SEQ ID NO:23 and/or the probe of sequence SEQ ID NO:34;
h) GP5 gene by using the primers of sequences SEQ ID NO:24 and SEQ ID NO:25 and/or the probe of sequence SEQ ID NO:35;
i) NPR3 gene by using the primers of sequences SEQ ID NO:26 and SEQ ID NO:27 and/or the probe of sequence SEQ ID NO:36.

**Table 3: Primers of the biomarkers of the invention used in dPCR**

| **Primers** | **5'-3' nucleotide sequence** | **SEQ ID NO:** |
|---|---|---|
| MROH6 FP | AGTAGCGGTACGAGATTTTGTAGT | 10 |
| MROH6 RP | CTCTAATACGCCGAAACCGAAAC | 11 |
| HOXB4 FP | GGGTTTGCGTTTTGTTTAGC | 12 |
| HOXB4 RP | TTACCTCGCTCTCTCCGAAA | 13 |
| chr7:129425301 FP | TTTGTACGTCGTCGGTTTTG | 14 |
| chr7:129425301 RP | ACGCCCAAACAAACAACAA | 15 |
| chr20:1784267 FP | GTATCGGGAATTAGGGGGAC | 16 |
| chr20:1784267 RP | CGTTAAAACCGAATCAAACGA | 17 |
| OPLAH FP | CGGGGTCGTTTTCGTAAGTA | 18 |
| OPLAH RP | GACCTCCTAAACCCGACGAT | 19 |
| CRTC1 FP | GTCGAGCGTGTTCGTAAAGATAC | 20 |
| CRTC1 RP | ACAAACAAAAAACCCCTCCGA | 21 |
| KCNQ4 FP | TTAGAAGTTTTCGTTTTCGGGTTC | 22 |
| KCNQ4 RP | AAAACGACGAAATCACAACCA | 23 |
| GP5 FP | CGCGAACGTTTTTGTGTG | 24 |
| GP5 RP | GCGCAAAAACCCGAACAC | 25 |
| NPR3 FP | CGTTTGGAATGTGAGTGGTG | 26 |
| NPR3 RP | CTAACCCGACGCCTAACTCA | 27 |

**Table 4: Probes of the biomarkers of the invention (FAM stands for Fluorescein Amidite, MGB stands for "Minor groove Binder"; NFQ stands for "Non-Fluorescent Quencher")**

| **Genes** | **Probes** | **SEQ ID NO:** |
|---|---|---|
| MROH6 | FAM-AACTCCGACTAAAACTCCG-MGB-NFQ | 28 |
| HOXB4 | FAM-CGCGCAAATTACGACTAAAA-MGB-NFQ | 29 |
| chr7:129425301 | FAM-ACGCCGCGACAAAAA-MGB-NFQ | 30 |
| chr20:1784267 | FAM-ACCCGCGTCAACTACAAT-MGB-NFQ | 31 |
| OPLAH | FAM-CTCATAAACGCTACCGTACTC-MGB-NFQ | 32 |
| CRTC1 | FAM-TCCGAAATTCTACAAACCCC-MGB-N FQ | 33 |
| KCNQ4 | FAM-ACCTACCCCGCGCAC-MGB-NFQ | 34 |
| GP5 | FAM-CGCGCACAATAAAATCCC-MGB-NFQ | 35 |
| NPR3 | FAM-CTCGAAAAACTTTACCGAAA-MGB-N FQ | 36 |

In a preferred embodiment, the level of methylation is determined by Next Generation Sequencing (NGS), by quantitative PCR (qPCR) or digital PCR (dPCR). The level of methylation of the biomarkers (a) to (i) is preferably determined by dPCR.

As used herein, the term "dPCR" stands for "digital PCR" and can be used interchangeably with the term "ddPCR" stands for "droplet-digital PCR". It is a refinement of conventional PCR methods wherein the sample is separated into a large number of partitions and the PCR reaction is carried out in each partition individually. This separation allows a more reliable collection and sensitive measurements of nucleic acid amounts. The method has been demonstrated as useful for studying variations in gene sequences. More precisely, the PCR solution is divided into smaller reactions through a water oil emulsion technique, which are then made to run PCR individually. For example, the PCR sample can be partitioned into pico to nanoliter size samples and encapsulated into oil droplets. Depending of the system used, from 5-10 million picoliter droplets to 20,000 oil nanoliter droplets can be created.

The present diagnostic method can use methods for detecting gene expression, for example dPCR, that use fluorogenic probes to improve the specificity and the sensitivity of the detection of the PCR products accumulated during PCR. Such assays are for example TaqMan gene expression assays using probes containing minor groove binding (MGB) moiety that enhances the Tm differential between matched and mismatched probes. In addition, these MGB probes may contain a non-fluorescent quencher (NFQ) that enhances spectral resolution when using multiple dyes in a reaction.

The probes used in a preferred embodiment are described in Table 4.

The method of the invention also comprises the step of comparison of the level of methylation of the biomarkers with the methylation level of the same biomarkers that is determined in a reference sample.
If at least one to nine of the biomarkers is (are) hypermethylated in the biological sample of the tested subject as compared to the same biomarkers in the reference sample, then the tested subject has a lung cancer or has a high risk to develop a lung cancer or to relapse of lung cancer.
In an embodiment, the biological samples according to the disclosure are body effluent or solid tissue such as biopsy specimen. The body effluent collected from patient is urine, stools, coughing up, plasma or blood sample, preferably blood sample.

In the method of the disclosure, the level of methylation of at least two genes is determined simultaneously or sequentially in the biological sample of the patient.

In another aspect, the biomarkers of the invention are used in methods to monitor and/or predict the outcome of lung cancer patients. These methods of monitoring and prediction are used by the oncologist to select appropriate treatments for maximizing the survival of patients. Appropriate treatments are for example chemotherapy, immunotherapy, radiotherapy, and/or surgery. The chemotherapeutic agent is selected in the list consisting in: an alkylating agent, an antimetabolite, a topoisomerase inhibitor, a platin based component, a specific kinase inhibitor, a hormone, a cytokine, an antiangiogenic agent, an antibody, an immunotherapy or a vascular disrupting agent.
The present disclosure relates to a method for monitoring the evolution of lung cancer in a patient suffering thereof comprising:
a) at a first time point, assessing the level of methylation in a biological sample of the patient of at least one gene or at least one nucleotide region selected in the groups consisting of:
   i) MROH6 gene, HOXB4 gene, OPLAH gene, CRCT1 gene, KCNQ4 gene, GP5 gene, NPR3 gene, chr7:129425301 gene and chr20:1784267 gene; or
   ii) the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene; or
   iii)
      a) MROH6 gene by using the primers of sequences SEQ ID NO:10 and SEQ ID NO:11 and/or the probe of sequence SEQ ID NO:28;
      b) HOXB4 gene by using the primers of sequences SEQ ID NO:12 and SEQ ID NO:13 and/or the probe of sequence SEQ ID NO:29;
      c) chr7:129425301 gene by using the primers of sequences SEQ ID NO:14 and SEQ ID NO:15 and/or the probe of sequence SEQ ID NO:30;
      d) chr20:1784267 gene by using the primers of sequences SEQ ID NO:16 and SEQ ID NO:17 and/or the probe of sequence SEQ ID NO:31
      e) OPLAH gene by using the primers of sequences SEQ ID NO:18 and SEQ ID NO: 19 and/or the probe of sequence SEQ ID NO:32;
      f) CRCT1 gene by using the primers of sequences SEQ ID NO:20 and SEQ ID NO:21 and/or the probe of sequence SEQ ID NO:33;
      g) KCNQ4 gene by using the primers of sequences SEQ ID NO:22 and SEQ ID NO:23 and/or the probe of sequence SEQ ID NO:34;
      h) GP5 gene by using the primers of sequences SEQ ID NO:24 and SEQ ID NO:25 and/or the probe of sequence SEQ ID NO:35;
      i) NPR3 gene by using the primers of sequences SEQ ID NO:26 and SEQ ID NO:27 and/or the probe of sequence SEQ ID NO:36;
      and
b) at a second time point, assessing the level of methylation of the gene(s) selected in the first time point (a) in a biological sample of the patient;
c) comparing the level of methylation of the selected gene(s) or nucleotide region between the second time point (b) and the first time point (a) or between the second time point (b) and a reference value.
The level of methylation in the method for monitoring the progress of lung cancer in a patient is also determined by a combination of at least two biomarkers, three biomarkers, four biomarkers, five biomarkers, six biomarkers, seven biomarkers, eight biomarkers or nine biomarkers selected in the groups consisting of:
i) MROH6 gene, HOXB4 gene, OPLAH gene, CRCT1 gene, KCNQ4 gene, GP5 gene, NPR3 gene, chr7:129425301 gene and chr20:1784267 gene; or
ii) the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene; or
iii)
   a) MROH6 gene by using the primers of sequences SEQ ID NO:10 and SEQ ID NO:11 and/or the probe of sequence SEQ ID NO:28;
   b) HOXB4 gene by using the primers of sequences SEQ ID NO: 12 and SEQ ID NO:13 and/or the probe of sequence SEQ ID NO:29;
   c) chr7:129425301 gene by using the primers of sequences SEQ ID NO:14 and SEQ ID NO:15 and/or the probe of sequence SEQ ID NO:30;
   d) chr20:1784267 gene by using the primers of sequences SEQ ID NO:16 and SEQ ID NO:17 and/or the probe of sequence SEQ ID NO:31
   e) OPLAH gene by using the primers of sequences SEQ ID NO:18 and SEQ ID NO:19 and/or the probe of sequence SEQ ID NO:32;
   f) CRCT1 gene by using the primers of sequences SEQ ID NO:20 and SEQ ID NO:21 and/or the probe of sequence SEQ ID NO:33;
   g) KCNQ4 gene by using the primers of sequences SEQ ID NO:22 and SEQ ID NO:23 and/or the probe of sequence SEQ ID NO:34;
   h) GP5 gene by using the primers of sequences SEQ ID NO:24 and SEQ ID NO:25 and/or the probe of sequence SEQ ID NO:35;
   i) NPR3 gene by using the primers of sequences SEQ ID NO:26 and SEQ ID NO:27 and/or the probe of sequence SEQ ID NO:36.

In the present method for monitoring, the sample in the first time point (a) is assessed prior to treatment for lung cancer of the patient suffering thereof and the sample in the second time point (b) is obtained after treatment for lung cancer of the patient suffering thereof.
It can be concluded that the malignancy of the lung cancer is worsening if the level of methylation in step (b) is significantly higher than the level determined in step (a). If step (a) is achieved prior to the treatment of the patient and step (b) is achieved after said patient has finalized his treatment for lung cancer, thus the efficiency of the treatment can be assessed.

In another aspect, the method for monitoring the progress of lung cancer also concerns patient who are treated against lung cancer before step (a) and before step (b).

Response to a treatment is defined for example according to RECIST 1.1 criteria (Mandard et al.). A Complete Response (CR) is defined as a disappearance of all target lesions. Any pathological lymph nodes (whether target or non-target) must have reduction in short axis to <10mm. A Partial Response (PR) is defined as at least 30% decrease in the sum of diameters of target lesions, taking as reference the baseline sum diameters. A Progressive Disease (PD) is defined as at least 20% increase in the sum of diameters of target lesions, taking as reference the smallest sum on study (this includes the baseline sum if that is the smallest on study). In addition to the relative increase of 20%, the sum must also demonstrate an absolute increase of at least 5mm. A Stable Disease (SD) is defined as neither sufficient shrinkage to qualify PR nor sufficient increase to qualify for PD, taking as reference the smallest sum diameters while on study. Time of the evaluation often depends of the disease.

If the methylation of at least one biomarker or a combination of biomarkers of the present disclosure has a lower level of methylation in step (b) than the level of methylation in step (a) as compared to the same biomarker(s), thus the patient responds efficiently to the tested treatment. Therefore, this decrease of the level of methylation is indicative of a responder's patient to the treatment against lung cancer. A responder is defined in the Complete Response RECIST 1.1 criteria.

On the contrary, if the methylation level of at least one biomarker or a combination of biomarkers of the present disclosure is equivalent or higher in the biological sample acquired after the administered treatment as compared to the same biomarker(s) in a sample acquired before the administered treatment, then the tested patient does not efficiently respond to the treatment against lung cancer. Therefore, the equivalent or higher level of methylation in step (b) than the level of methylation in step (a) is indicative of a non-responder's patient to the treatment against lung cancer. As used herein, a "non-responder" is considered as a patient with a progressive disease or a stable disease as defined according to RECIST 1.1 criteria.

In the method for monitoring the evolution of lung cancer, the level of methylation of at least two genes is determined simultaneously or sequentially in the biological sample of the patient.

As used herein, the reference value according to the present disclosure is obtained in a sample from a healthy donor or in a sample from a patient suffering from lung cancer previous to a treatment or performed in an earlier time of the treatment.

The present invention also relates to a method for adapting the therapeutic regimen for a patient suffering from lung cancer. In this method, the treatment for lung cancer of the patient suffering thereof is adapted in a final step (c) on the basis of the comparison of steps (a) and (b) as described in the method for monitoring the evolution of lung cancer.

The present disclosure relates to a method for predicting the clinical outcome of a patient suffering of lung cancer comprising the step of:
a) assessing the level of methylation in a biological sample of the patient of at least one gene or nucleotide region selected in one of the groups consisting of :
   i) MROH6 gene, HOXB4 gene, OPLAH gene, CRCT1 gene, KCNQ4 gene, GP5 gene, NPR3 gene, chr7:129425301 gene and chr20:1784267 gene; or
   ii) the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene; or
   iii)
      a) MROH6 gene by using the primers of sequences SEQ ID NO:10 and SEQ ID NO:11 and the probe of sequence SEQ ID NO:28;
      b) HOXB4 gene by using the primers of sequences SEQ ID NO:12 and SEQ ID NO:13 and the probe of sequence SEQ ID NO:29;
      c) chr7:129425301 gene by using the primers of sequences SEQ ID NO:14 and SEQ ID NO:15 and the probe of sequence SEQ ID NO:30;
      d) chr20:1784267 gene by using the primers of sequences SEQ ID NO:16 and SEQ ID NO:17 and the probe of sequence SEQ ID NO:31
      e) OPLAH gene by using the primers of sequences SEQ ID NO:18 and SEQ ID NO: 19 and the probe of sequence SEQ ID NO:32;
      f) CRCT1 gene by using the primers of sequences SEQ ID NO:20 and SEQ ID NO:21 and the probe of sequence SEQ ID NO:33;
      g) KCNQ4 gene by using the primers of sequences SEQ ID NO:22 and SEQ ID NO:23 and the probe of sequence SEQ ID NO:34;
      h) GP5 gene by using the primers of sequences SEQ ID NO:24 and SEQ ID NO:25 and the probe of sequence SEQ ID NO:35;
      i) NPR3 gene by using the primers of sequences SEQ ID NO:26 and SEQ ID NO:27 and the probe of sequence SEQ ID NO:36;
      and
b) comparing the level of methylation of the selected gene(s) to a reference value;
c) predicting the clinical outcome on the basis of the comparison methylation levels of step b).
If at least one biomarker as well as a combination of two biomarkers, three biomarkers, four biomarkers, five biomarkers, six biomarkers, seven biomarkers, eight biomarkers or nine biomarkers is (are) hypermethylated in the biological sample of the patient as compared to the same biomarker(s) in the reference sample, then the patient is likely to have a bad clinical outcome. On the contrary, if at least one biomarker as well as a combination of two biomarkers, three biomarkers, four biomarkers, five biomarkers, six biomarkers, seven biomarkers, eight biomarkers or nine biomarkers is (are) not significantly hypermethylated in the biological sample of the patient as compared to the same biomarker(s) in the reference sample, then the patient is likely to have a good clinical outcome.

An array comprising at least one of the probes set forth in Table 4.

A kit comprising primers targeting at least one of the gene selected in the group consisting of: MROH6 gene, HOXB4 gene, OPLAH gene, CRCT1 gene, KCNQ4 gene, GP5 gene, NPR3 gene, chr7:129425301 gene and chr20:1784267 gene, preferably the primers set forth in Table 4.

The present invention is illustrated by the following Figures and Examples, without being limited thereby:

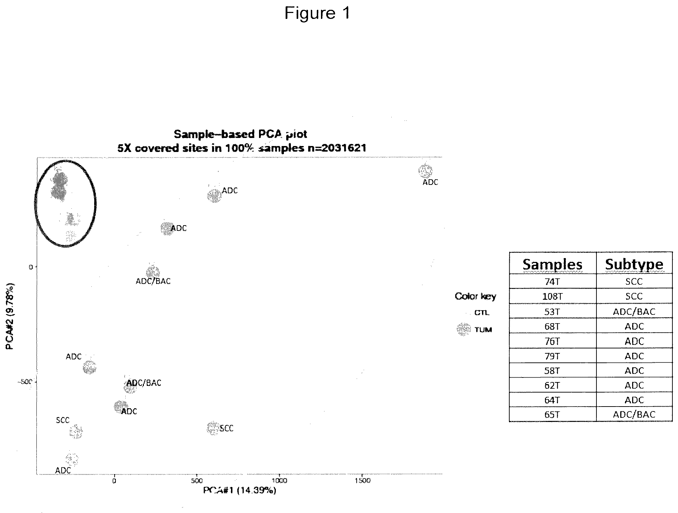

Figure 1: Principal Component Analysis (all the biomarkers 180 897 DMRs). Figure 1 discloses the principal component analysis of Methyl-seq based DNA methylation data from NSCLC tumor tissue (n=10) and paired adjacent non-tumor tissue (n=10). DNA methylome libraries were prepared with the use of SeqCap Epi CpGiant Enrichment kit (Roche) and the samples were passed to the NextseqTM 500 sequencer (Illumina). Plot of principal component analysis reveals a clear difference between tumor samples (large round) and healthy controls (small round) based on their methylation profiles.

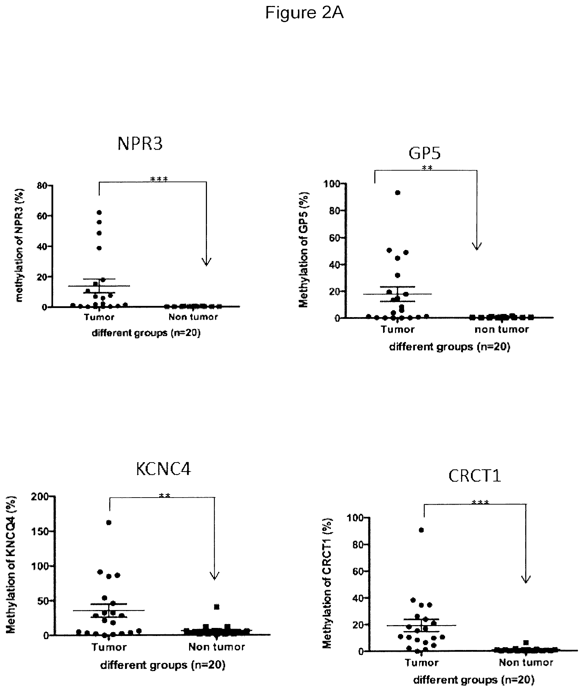

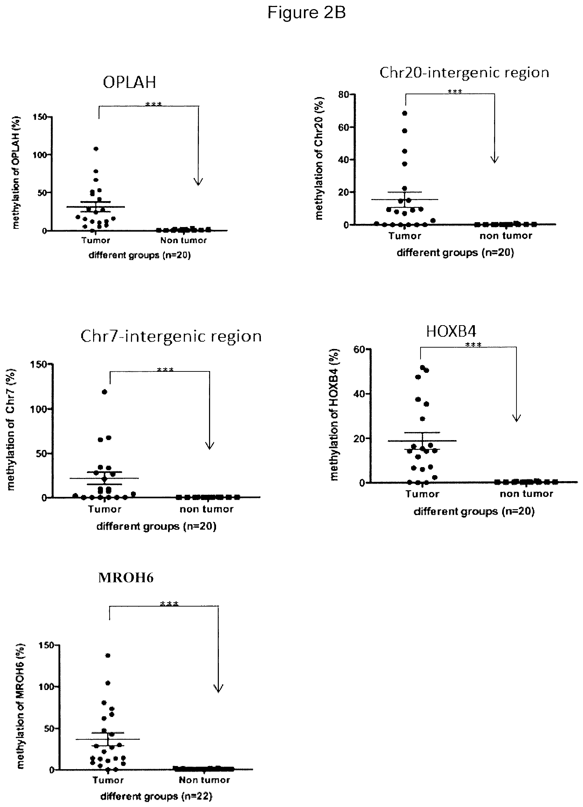

Figure 2: DNA methylation of candidate biomarkers in lung tumor tissue by ddPCR. Figure 2 discloses the methylation level of selected biomarkers in tumor and adjacent non-tumor tissue DNA (first cohort) (n= 20-22) from lung cancer patients by ddPCR and their detection sensitivity and specificity for each biomarker. The number of the samples run for each biomarker depended on the availability of DNA. Paired non-parametric Wilcoxon test was used for the analysis of the hypermethylation difference between normal and adjacent tissue DNA. The sum of the average and standard deviation of methylation level in non-tumor tissue DNA was set as the threshold for calculating sensitivity and specificity of each selected biomarker.

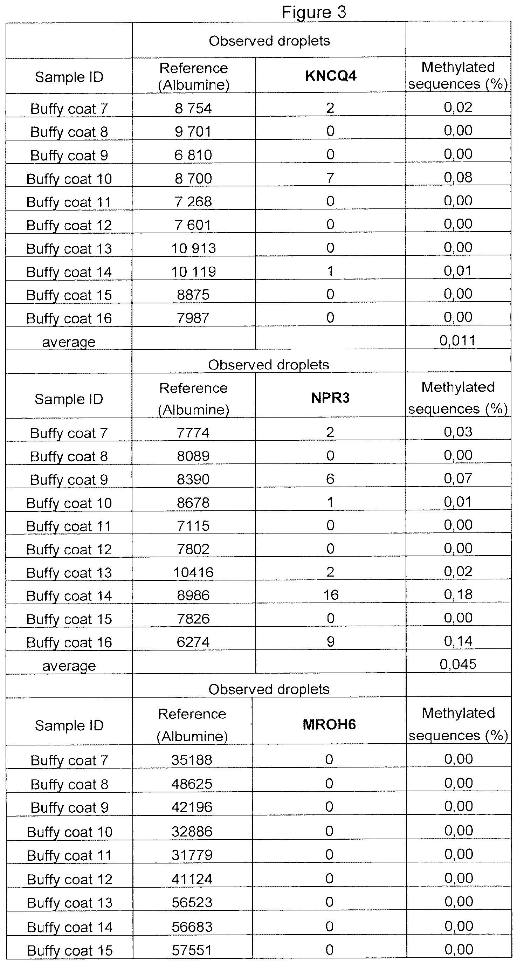

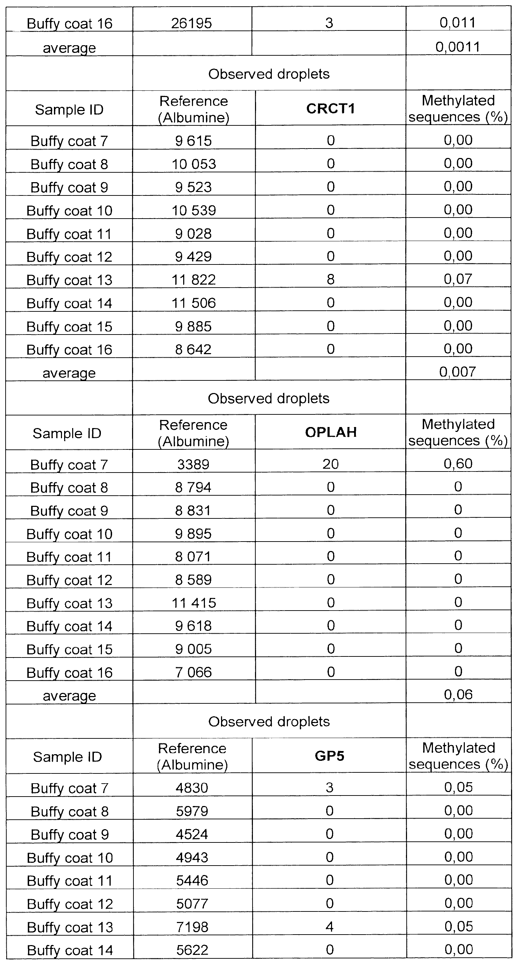

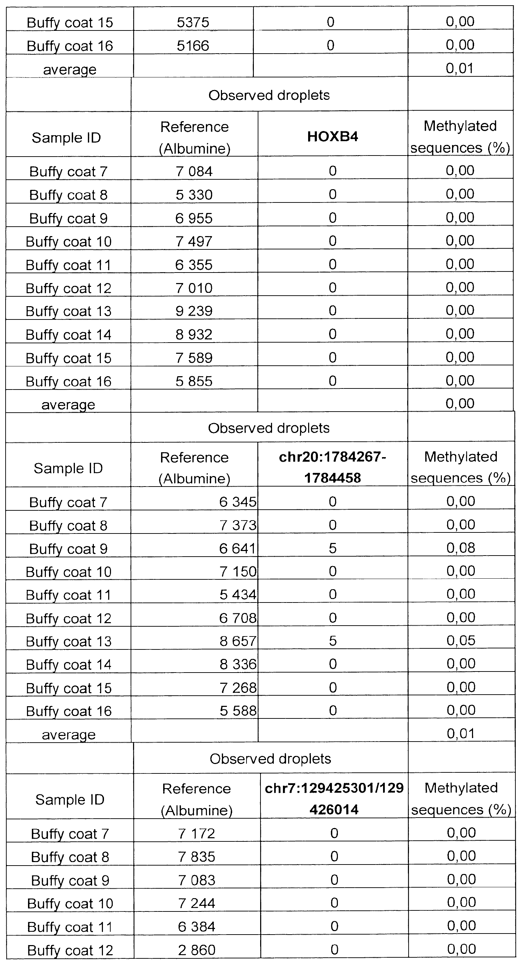

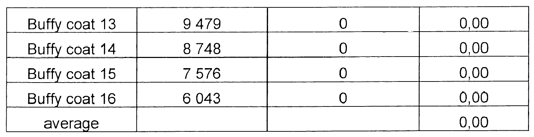

Figure 3: Methylation profiles of candidate biomarkers in buffy coat DNA by ddPCR. Figure 3 discloses the methylation level of the selected biomarkers in healthy buffy coat DNA measured by ddPCR. Buffy coat from healthy individuals (n=10) were purchased from Biopredic Internal. DNA was extracted with the use of QIAmp Circulating Nucleic Acid Kit (Qiagen). Methylation level of the selected biomarkers in buffy coat was measured by ddPCR.

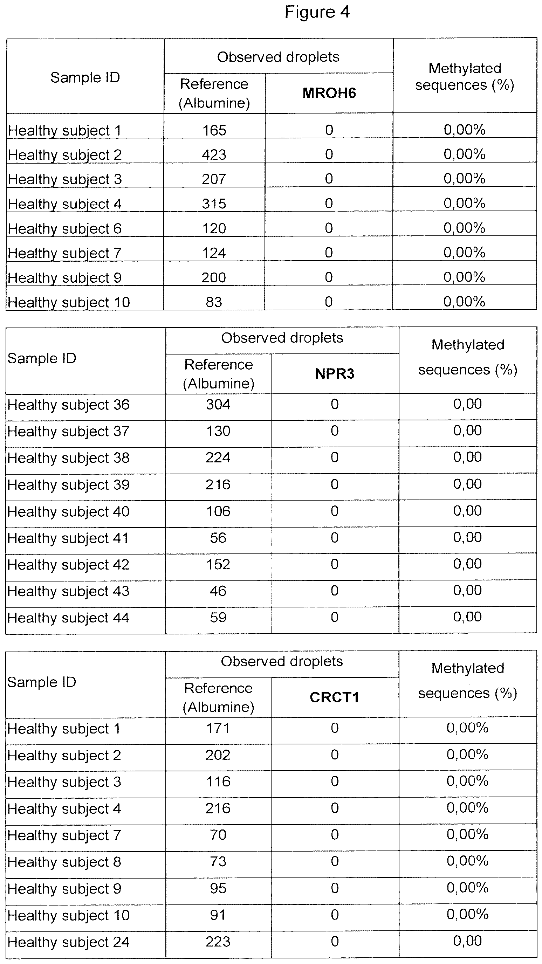

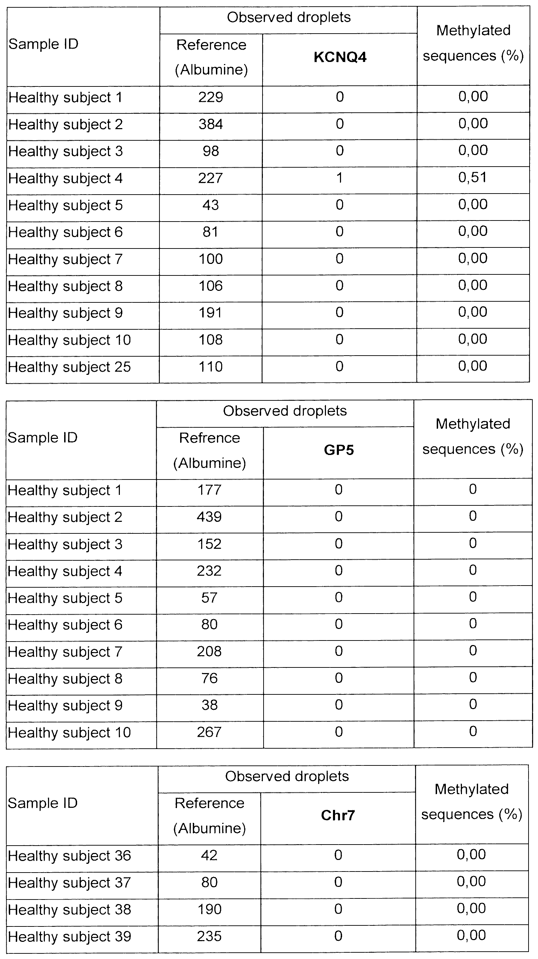

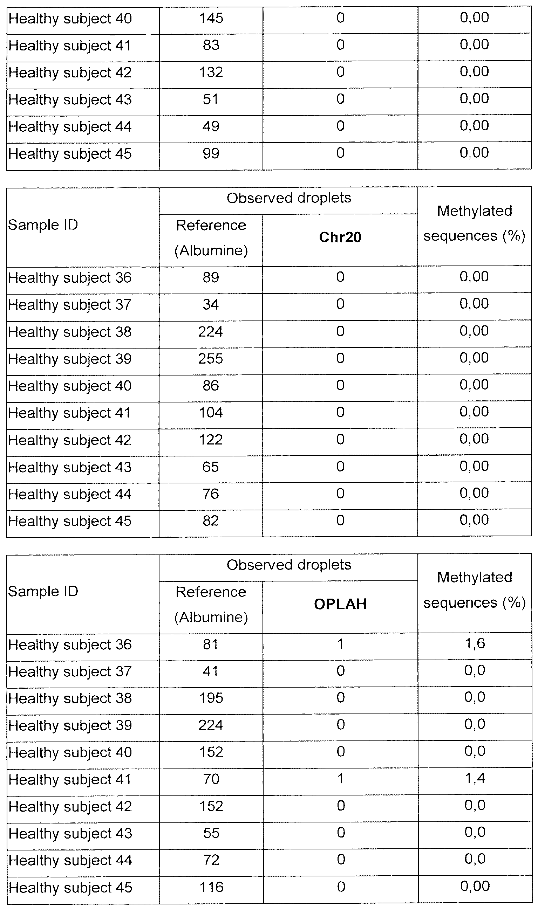

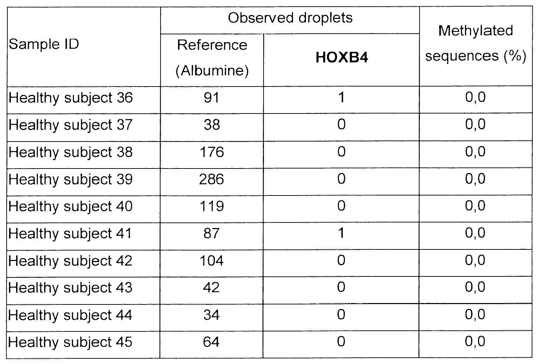

Figure 4: Methylation profiles of candidate biomarkers in healthy plasma by ddPCR. Figure 4 discloses the methylation level of the selected biomarkers in healthy plasma circulating cell free (ccfDNA) measured by ddPCR. Healthy plasma was purchased from Biopredic Internal. Circulating cell free DNA was extracted with the use of *Maxwell*^{®} RSC *ccfDNA Plasma Kit.* Methylation level of the selected biomarkers in plasma ccfDNA (n=8-11, depending on the availability of ccfDNA) was measured by ddPCR.

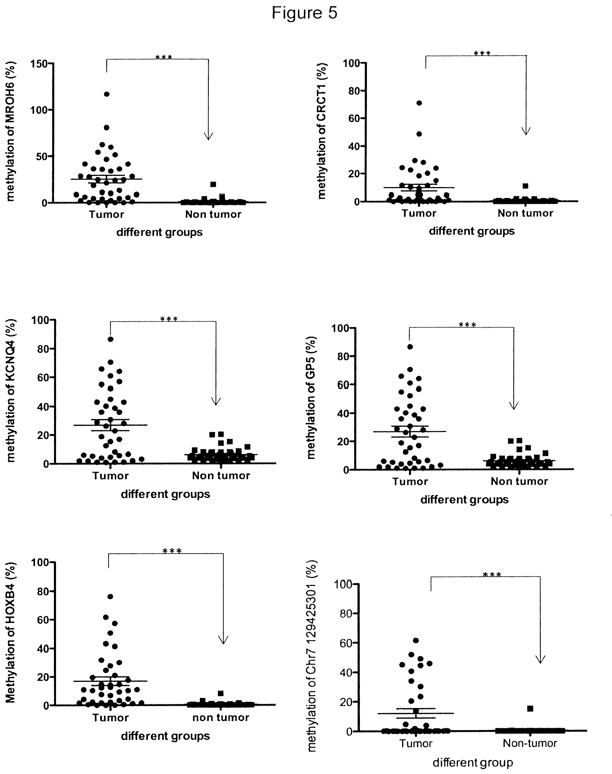

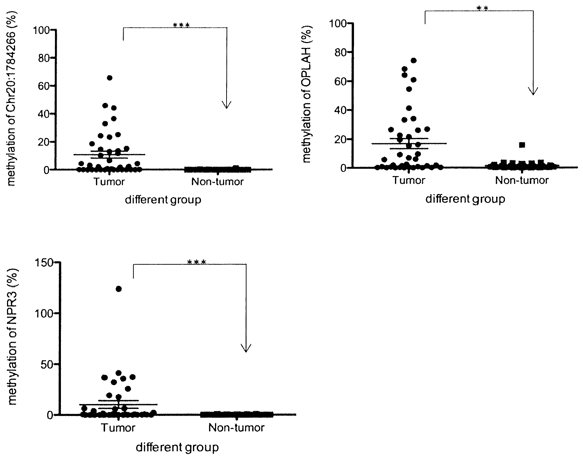

Figure 5: DNA methylation of candidate biomarkers in lung tumor tissue by ddPCR. Figure 5 discloses the methylation level of selected biomarkers in 2^{nd} cohort of tumor (n=39) and adjacent non-tumor tissue DNA (n= 38) from lung cancer patients by ddPCR and their detection sensitivity and specificity for each biomarker.
Mann-whitney test was used for the analysis of the hypermethylation difference between normal and adjacent tissue DNA. The sum of the average and standard deviation of methylation level in non-tumor tissue DNA of the first cohort was used as the threshold for calculating sensitivity and specificity of each selected biomarker.

### 1. Materials and Methods

### Patients and healthy subjects

Matched tumor and adjacent non-tumor tissue biopsies from 23 non-small lung cancer (NSCLC) patients were tested for ddPCR validation tests (59.0±11.5 years old; T1-T4 stages) including those used for Methyl-sequencing (MethylSeq) comprising tissues from 10 non-small lung cancer (NSCLC) patients (57.0±9.6 years old; T1-T4 stages).
Tissues used for ddPCR were obtained from 23 non-small lung cancer (NSCLC) patients including 12 adenocarcinoma (ADC), 3 adenocarcinoma/bronchioalveolar (ADC/BAC), 3 Large Cell Carcinoma (LCC), 2 bronchioalveolar (BAC) and 3 squamous cell carcinoma (SCC) patients. Except 8 females, the other 15 patients are male.
Tissues used for MethylSeq were obtained from 10 non-small lung cancer (NSCLC) patients including 6 adenocarcinoma (ADC), 2 adenocarcinoma/bronchioalveolar (ADC/BAC) and 2 squamous cell carcinoma (SCC) patients. Except one female, the other 9 patients are male.
Plasma samples and buffy coat from healthy individuals were sourced from Biopredic International (Saint Grégoire, France).
This study is approved by the local ethics committee and informed written consent was obtained from all the patients.

### Sample preparation, storage, DNA extraction and quantification

### • Tumor

Tumor and adjacent non-tumor tissue biopsies were flash frozen in liquid nitrogen immediately after resection until further analysis. Each tumor was reviewed by a pathologist and the tumor cell content was assessed by hematoxylin-eosin-safran staining. DNA was extracted with the QIAampDNAMini Kit (Qiagen) according to the manufacturer's instructions. DNA concentration was measured by Qubit 2.0 fluorometer (Invitrogen, Life Technologies) with the use of the dsDNA BR Assay (Invitrogen). Extracted DNA samples were stored at -20 °C before testing.

### • Plasma

Plasma samples of healthy individuals were received in dry ice, aliquoted and immediately frozen at -80 °C. Before extraction, plasma samples were centrifuged at 3000g for 10 min and then extracted with the use of the QIAmp Circulating Nucleic Acid Kit (Qiagen) or the ccfDNA Plasma kit (Promega) by RSC Maxwell instrument according to the manufacturer's instructions. The quantity of DNA was measured by Qubit 2.0 fluorometer (Invitrogen, Life Technologies) with the use of the dsDNA HS Assay (Invitrogen). Extracted DNA samples were stored at -20 °C before testing.

### • Buffy coat

Buffy coat was prepared from whole blood of healthy subjects and then extracted with the use of the QIAmp Circulating Nucleic Acid Kit (Qiagen) according to the manufacturer's instructions. The quantity of DNA was measured by Qubit 2.0 fluorometer (Invitrogen, Life Technologies) with the use of the dsDNA BR Assay (Invitrogen). Extracted DNA samples were stored at - 20 °C before testing.

### Tumor tissue DNA, buffy coat DNA, Plasma ccfDNA bisulfite conversion

Tumor DNA, buffy coat DNA or plasma DNA was modified by bisulfite using the EZ DNA Methylation-Gold Kit (Zymo Research). In brief, bisulfite reaction was carried out in a thermocycler at 98 °C for 12 min and 64 °C for 2h35 min. The cleanup of bisulfite-converted DNA followed the recommendations of the manufacturer and converted DNA was eluted in M-Elution Buffer and stored at -20 °C.

### Genome-wide DNA methylome profiling in lung cancer patients by Methyl-Seq

DNA methylome libraries were prepared with the use of SeqCap Epi CpGiant Enrichment kit (Roche) according to the manufacturer' instructions. 500ng to 1µg of tumor or non-tumor tissue DNA or buffy coat DNA or plasma DNA was fragmented with the use of Bioruptor (Diagenode s.a, Belgium). NGS adapters (Next Generation Sequencing adapters) were ligated to the fragmented DNA followed by the bisulfite conversion treatment with the use of EZ DNA Methylation-Lightning Kit (Zymo research). After several steps of purification and amplification, bisulfite-converted samples were hybridized with SeqCap Epi probe pool and then captured by SeqCap Pure Capture Beads. Post capture amplification was applied before passing the samples to the NextseqTM 500 sequencer (Illumina).

### Analysis and identification of DNA methylation biomarkers based on Methyl-Seq data

Following a quality control, sequence data was aligned to bisulfite converted reference genome and analyzed for Differentially Methylated CpG sites (DMC) and Differentially Methylated Regions (DMR) between tumor and non-tumor DNA with the use of Metilene: a R script. DMRs are the regions containing at least 5 DMCs in more than 80% of the samples. A list of DMRs was generated including different information: the level of methylation in non-tumor tissue DNA, the level of methylation in tumor tissue DNA, the difference of methylation level between tumor and non-tumor tissue DNA, q-value, gene symbol corresponding to the DMRs identified, the position of DMRs inside the chromosome. Based on this list of DMR generated, different parameters were then used for identifying DNA methylation biomarkers for lung cancer patients. First, all the DMRs with a q-value less than 0.05 were selected. Second, DMRs with a difference of methylation level more than 20% between tumor and non-tumor DNA and a methylation level in non-tumor DNA less than 5% were chosen. Meanwhile, DMRs with a difference of methylation level more than 30% between tumor and non-tumor DNA and a methylation level in non-tumor DNA less than 10% were also chosen. Following this, DMRs were removed from the candidate biomarkers list if (a) the methylation level of the DMRs in buffy coat DNA was more than 5%, (b) the DMRs were present in the intergenic region or (c) the DMRs were already described in a public data.

### Detection and measurement of methylation level of the selected biomarkers by droplet-based digital PCR (ddPCR) in tumor, buffy coat DNA and plasma ccfDNA

The methylation level of the selected biomarkers in tumor, paired non-tumor, buffy coat DNA and plasma ccfDNA is tested by ddPCR (Bio-Rad's QX200 ddPCR System). Duplex format is used to analyze hypermethylation of each biomarker with albumin for normalizing the input DNA amount.

In one aspect, the invention provides the primers pair SEQ ID NOs:10-11 and the probe SEQ ID NO:28 for MROH6 biomarker, the primers pair SEQ ID NOs:12-13 and the probe SEQ ID NO:29 for HOXB4 biomarker, the primers pair SEQ ID NOs:14-15 and the probe SEQ ID NO:30 for chr7:129425301 biomarker, the primers pair SEQ ID NOs:16-17 and the probe SEQ ID NO:31 for chr20:1784267 biomarker, the primers pair SEQ ID NOs:18-19 and the probe SEQ ID NO:32 for OPLAH biomarker, the primers pair SEQ ID NOs:20-21 and the probe SEQ ID NO:33 for CRTC1 biomarker, the primers pair SEQ ID NOs:22-23 and the probe SEQ ID NO:34 for KCNQ4 biomarker, the primers pair SEQ ID NOs:24-25 and the probe SEQ ID NO:35 for GP5 biomarker and the primers pair SEQ ID NOs:26-27 and the probe SEQ ID NO:36 for NPR3 biomarker are used.

11µL ddPCR Supermix(No dUTP) (Bio-Rad laboratories) is mixed with 1.1µ L 20x Assay Mix containing 8µM of forward and reverse primers, 4µM of 6-FAM probe targeting the biomarker and 12µM of VIC Taqman^{®} labeled-probes targeting Albumin, and bisulfite treatment modified DNA template to a final reaction volume of 22µL. A minimum of 10ng of modified DNA is used in each reaction. Droplets are then generated followed by PCR amplification.

A limit of blank (LOB) with the use of negative control: normal control genomic DNA has been calculated as described by Taly et al. 2013. It is defined by the frequency of positive droplets measured in normal control DNA samples with no hyper-methylated DNA present (n=10). The calculated LOB was subtracted from each sample for calculating their methylation level.

Samples were considered positive when the number of observed droplets was higher than LOB value. The methylation level of each sample was calculated as the ratio of the copy number of methylated sequences of each biomarker over the copy number of albumin sequences detected.

Two DNA controls were used for ensuring the proper realization of the modification treatment (Positive control: universal hypermethylated DNA (Zymo research) and negative control: normal human genomic DNA) (Promega).

### Calculation of detection sensitivity and specificity

The sum of the average and standard deviation of methylation level in non-tumor tissue DNA is used as the threshold for calculating sensitivity and specificity of each selected biomarker. Sensitivity is the percentage of the patients showing higher methylation level in tumor tissues than the defined threshold. Specificity is the percentage of the patients showing lower methylation level in non-tumor tissues than the defined threshold.

### Statistical analysis

Statistical analysis was performed using Prism Software (GraphPad Software Inc.). For the analysis of the hypermethylation difference between normal and adjacent tissues, paired non parametric Wilcoxon test is used. Methyl-Seq data are visualized and classified using principal component analysis (PCA) tool included in the R script (metilene).

### 2. Results

### 2.1 Identification workflow of DNA methylation biomarkers

### Identification of DNA methylation biomarkers based on Methyl-Seq data

For a first large scale screening, a total of 20 samples (paired tumor and non-tumor tissue DNA) from 10 non-small cell lung cancer (NSCLC) patients are analyzed for methylome profiles by Methyl-Seq following SeqCap Epi CpGiant Enrichment libraries preparation. Thereafter, a Principal Component Analysis (PCA) of methylation profiles in lung tumor tissue DNA and matched healthy tissue DNA was performed (Figure 1). There was a clear difference in methylation profiles between the tissue DNAs from tumor and non-tumor tissues separating the samples into two major clusters. As described in the workflow, 180 897 DMRs are identified in lung tumor tissue DNA compared with non-tumor tissue DNA. Among them, 16 653 DMRs presented a q-value lower than 0.05. Following this first step, a second threshold (difference of methylation level more than 20% between tumor DNA and non-tumor DNA; and methylation level less than 5% in non-tumor DNA) is applied for the identification of potential DNA methylation biomarkers for lung cancer and 197 DMRs are selected. Meanwhile, a difference of methylation level more than 30% between tumor DNA and non-tumor DNA and a methylation level less than 10% in non-tumor DNA are applied for the identification of potential DNA methylation biomarkers for lung cancer and 80 DMRs are subsequently selected. After removing the common ones, 267 biomarkers were left.

### Filtering of the DNA methylation biomarkers based on buffy coat sequencing data

The final usage of these identified biomarkers is to precisely detect and quantify circulating tumor DNA (ctDNA) within plasma ccfDNA. Such identification could be complicated by its dilution within ccfDNA, which could be exacerbated by pre-analytic issues (including but not limited to blood collection and handling, plasma preparation, plasma storage, DNA extraction, DNA storage). In particular, improper blood and plasma handling and preparation could lead to lysis of blood cells releasing DNA into plasma and further diluting the ctDNA fraction and complicating analysis. Moreover, the presence of identified methylation markers within blood cell DNA could lead to false positive detection of ctDNA in plasma or overestimation of the ctDNA fraction. Consequently, a Methyl-Seq analysis was performed on DNA extracted from buffy coat fraction from normal subjects to remove the potential biomarkers showing more than 5% methylation in buffy coat DNA. Following this step, 223 DMRs markers were selected from the previous 267 DMRs.

### Additional filtering steps

Subsequently, a serie of steps based on defined criteria (including difference between the methylation fraction within Tumor and Non-Tumor tissues, the methylation level in non-tumor tissues, q-value, the calculated specificity and sensitivity of the marker based on first cohort analysis, the methylation level in Buffy Coat DNA are used to reduce the number of selected methylated biomarkers to 22.

### Droplet-based digital PCR assay development

A combination of primers and probes for each of these 22 biomarkers are designed and tested by RT-PCR and ddPCR. ddPCR assays are developed and validated with the use of commercialized positive control: universal hypermethylated DNA and negative control consisting of normal human whole blood genomic DNA. Some biomarkers out of the 22 selected biomarkers showed clear positive droplet cluster in negative control DNA, which gave a high limit of Background (LOB) of these assays and are thus subsequently removed from the final list. From the initial list of 22 markers, 15 markers are selected for further evaluation (Figure 2A-2B).

### Methylation profiles of the selected biomarkers in buffy coat DNA by ddPCR

To further refine the selection of markers an additional buffy coat analysis is performed using an orthogonal test (ddPCR) to remove the potential markers which showed high DNA methylation in buffy coat. Biomarkers showed a non-negligible methylation level in buffy coat DNA, which might cause false positive detection because of lysis of blood cells in patient's plasma during pre-analytic step are removed from the final list. Following this step 11 markers were selected for further evaluation (Figure 3).

### Methylation profiles of the selected biomarkers in plasma ccfDNA from healthy individuals by ddPCR

The purpose of these biomarkers is to detect DNA hypermethylation in lung cancer patients but not in healthy individuals. In order to validate this, the methylation level in plasma ccfDNA from healthy individuals was investigated by ddPCR. Biomarkers showing clearly detectable methylation level in healthy plasma ccfDNA, which could cause background noise when these biomarkers are applied on patient's plasma, are removed from the final list. Finally, based on all these different tests, 9 biomarkers were identified (MORH6, CRTC1, GP5, HOXB4, Chr7:129425301, Chr20:1784266, KCNQ4, NPR3 and OPLAH) and selected as the biomarkers for diagnostic and monitoring of lung cancer patients, especially non-small cell lung cancer patients (Figure 4).

### 2.2 Validation of the biomarkers on Independent Cohorts

The 9 biomarkers selected are evaluated by ddPCR on a second independent tissue cohort of 40 adenocarcinoma lung cancer (Figure 5). The 40 peri-tumoral tissues of these same patients are also studied to calculate and validate the sensitivity and specificity of each of the 9 biomarkers.

The best sensitivity is obtained for the marker HOXB4 with 92.3 % and the best specificity for the marker CRCT1 with 97.4 % (Table 5).

**Table 5: Sensitivity and specificity of markers**

| | CRCT1 | KCNQ4 | GP5 | HOXB4 | chr7 | chr20 | OPLAH | NPR3 | MROH6 |
|---|---|---|---|---|---|---|---|---|---|
| Sensitivity | 51.3 | 59 | 69.2 | 92.3 | 53.8 | 61.5 | 56.4 | 53.8 | 84.6 |
| Specificity | 97.4 | 92.1 | 94.7 | 78.9 | 94.7 | 94.7 | 78.9 | 86.8 | 86.8 |

The 9 biomarkers are evaluated once again by ddPCR on a third independent tissue cohort and a plasma cohort. Based on the results of the validation on a third cohort, 5 biomarkers were identified (MORH6, HOXB4, Chr7:129425301, Chr20:1784266 and OPLAH) and selected as the preferred biomarkers for diagnostic and monitoring of lung cancer patients.

## Claims

1. A method for diagnosing lung cancer or diagnosing relapse of lung cancer in a biological sample of a patient by determination of the level of methylation of at least one gene selected in the group consisting of : MROH6 gene, HOXB4 gene, OPLAH gene, CRCT1 gene, KCNQ4 gene, GP5 gene, NPR3 gene, chr7:129425301 gene and chr20:1784267 gene.

2. The method according to claim 1, wherein the level of methylation consisting in an hypermethylation is indicative of lung cancer.

3. The method according to claim 1 or 2 comprising the steps of:
c) assessing in a biological sample of a patient the level of methylation of at least one gene selected in the group consisting of: MROH6 gene, HOXB4 gene, OPLAH gene, CRCT1 gene, KCNQ4 gene, GP5 gene, NPR3 gene, chr7:129425301 gene and chr20:1784267 gene; and
d) comparing the level of methylation in (a) with the level of methylation for the same gene(s) in a control sample;
wherein the level of methylation in (a) superior to the level of methylation in (b) is indicative of lung cancer.

4. The method according to any one of claims 1 to 3, wherein the level of methylation is determined in at least one nucleotide region selected in the group consisting of: the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene, the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene.

5. The method according to any one of claims 1 to 4, wherein the level of methylation is determined in at least one the gene selected in the group consisting of:
a) MROH6 gene by using the primers of sequences SEQ ID NO:10 and SEQ ID NO:11 and the probe of sequence SEQ ID NO:28;
b) HOXB4 gene by using the primers of sequences SEQ ID NO:12 and SEQ ID NO:13 and the probe of sequence SEQ ID NO:29;
c) chr7:129425301 gene by using the primers of sequences SEQ ID NO:14 and SEQ ID NO:15 and the probe of sequence SEQ ID NO:30;
d) chr20:1784267 gene by using the primers of sequences SEQ ID NO:16 and SEQ ID NO:17 and the probe of sequence SEQ ID NO:31.
e) OPLAH gene by using the primers of sequences SEQ ID NO:18 and SEQ ID NO:19 and the probe of sequence SEQ ID NO:32;
f) CRCT1 gene by using the primers of sequences SEQ ID NO:20 and SEQ ID NO:21 and the probe of sequence SEQ ID NO:33;
g) KCNQ4 gene by using the primers of sequences SEQ ID NO:22 and SEQ ID NO:23 and the probe of sequence SEQ ID NO:34;
h) GP5 gene by using the primers of sequences SEQ ID NO:24 and SEQ ID NO:25 and the probe of sequence SEQ ID NO:35;
i) NPR3 gene by using the primers of sequences SEQ ID NO:26 and SEQ ID NO:27 and the probe of sequence SEQ ID NO:36;

6. The method according to any one of claims 1 to 5, wherein the level of methylation is determined by Next Generation Sequencing (NGS), quantitative PCR (qPCR) or digital PCR (dPCR).

7. The method according to any one of claims 1 to 6, wherein the biological sample is urine, stools, plasma or blood sample.

8. The method according to any one of claims 1 to 7, wherein the level of methylation of at least two genes is determined simultaneously or sequentially in the biological sample of the patient.

9. A method for monitoring the evolution of lung cancer in a patient suffering thereof comprising:
a) at a first time point, assessing the level of methylation in a biological sample of the patient of at least one gene selected in one of the groups consisting of:
i) MROH6 gene, HOXB4 gene, OPLAH gene, CRCT1 gene, KCNQ4 gene, GP5 gene, NPR3 gene, chr7:129425301 gene and chr20:1784267 gene; or
ii) the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene; or
iii)
a) MROH6 gene by using the primers of sequences SEQ ID NO:10 and SEQ ID NO: 11 and the probe of sequence SEQ ID NO:28;
b) HOXB4 gene by using the primers of sequences SEQ ID NO:12 and SEQ ID NO:13 and the probe of sequence SEQ ID NO:29;
c) chr7:129425301 gene by using the primers of sequences SEQ ID NO:14 and SEQ ID NO:15 and the probe of sequence SEQ ID NO:30;
d) chr20:1784267 gene by using the primers of sequences SEQ ID NO:16 and SEQ ID NO:17 and the probe of sequence SEQ ID NO:31
e) OPLAH gene by using the primers of sequences SEQ ID NO:18 and SEQ ID NO:19 and the probe of sequence SEQ ID NO:32;
f) CRCT1 gene by using the primers of sequences SEQ ID NO:20 and SEQ ID NO:21 and the probe of sequence SEQ ID NO:33;
g) KCNQ4 gene by using the primers of sequences SEQ ID NO:22 and SEQ ID NO:23 and the probe of sequence SEQ ID NO:34;
h) GP5 gene by using the primers of sequences SEQ ID NO:24 and SEQ ID NO:25 and the probe of sequence SEQ ID NO:35;
i) NPR3 gene by using the primers of sequences SEQ ID NO:26 and SEQ ID NO:27 and the probe of sequence SEQ ID NO:36;
and
b) at a second time point, assessing the level of methylation of the gene(s) selected in (a) in a biological sample of the patient;
c) comparing the level of methylation of the selected gene(s) between (b) and (a) or between (b) and a reference value.

10. The method according to claim 9, wherein the sample in (a) is assessed prior to treatment for lung cancer of the patient suffering thereof and the sample in (b) is obtained after treatment for lung cancer of the patient suffering thereof.

11. The method according to claim 9, wherein the patient has been treated against lung cancer before (a) and before (b).

12. The method according to any one of claims 9 to 11, wherein the lower level of methylation in (b) than the level of methylation in (a) is indicative of a responder's patient to the treatment against lung cancer.

13. The method according to any one claims 9 to 12, wherein the equivalent or higher level of methylation in (b) than the level of methylation in (a) is indicative of a non-responder's patient to the treatment against lung cancer.

14. The method according to any one of claims 9 to 13, wherein the level of methylation of at least two genes is determined simultaneously or sequentially in the biological sample of the patient.

15. The method according to any one of claims 9 to 14, wherein the reference value is obtained in a healthy donor.

16. The method according to any one of claims 9 to 15, wherein further the step (c) the treatment for lung cancer of the patient suffering thereof is adapted on the basis of the comparison of step (c).

17. A method for predicting the clinical outcome of a patient suffering of lung cancer comprising the step of:
a) assessing the level of methylation in a biological sample of the patient of at least one gene selected in one of the groups consisting of:
i) MROH6 gene, HOXB4 gene, OPLAH gene, CRCT1 gene, KCNQ4 gene, GP5 gene, NPR3 gene, chr7:129425301 gene and chr20:1784267 gene; or
ii) the nucleotide region of SEQ ID NO:1 in the MROH6 gene, the nucleotide region of SEQ ID NO:2 in the HOXB4 gene, the nucleotide region of SEQ ID NO:3 in the chr7:129425301 gene, the nucleotide region of SEQ ID NO:4 in the chr20:1784267 gene the nucleotide region of SEQ ID NO:5 in the OPLAH gene, the nucleotide region of SEQ ID NO:6 in the CRCT1 gene, the nucleotide region of SEQ ID NO:7 in the KCNQ4 gene, the nucleotide region of SEQ ID NO:8 in the GP5 gene and the nucleotide region of SEQ ID NO:9 in the NPR3 gene; or
iii)
a) MROH6 gene by using the primers of sequences SEQ ID NO:10 and SEQ ID NO: 11 and the probe of sequence SEQ ID NO:28;
b) HOXB4 gene by using the primers of sequences SEQ ID NO:12 and SEQ ID NO: 13 and the probe of sequence SEQ ID NO:29;
c) chr7:129425301 gene by using the primers of sequences SEQ ID NO:14 and SEQ ID NO:15 and the probe of sequence SEQ ID NO:30;
d) chr20:1784267 gene by using the primers of sequences SEQ ID NO:16 and SEQ ID NO:17 and the probe of sequence SEQ ID NO:31
e) OPLAH gene by using the primers of sequences SEQ ID NO:18 and SEQ ID NO: 19 and the probe of sequence SEQ ID NO:32;
f) CRCT1 gene by using the primers of sequences SEQ ID NO:20 and SEQ ID NO:21 and the probe of sequence SEQ ID NO:33;
g) KCNQ4 gene by using the primers of sequences SEQ ID NO:22 and SEQ ID NO:23 and the probe of sequence SEQ ID NO:34;
h) GP5 gene by using the primers of sequences SEQ ID NO:24 and SEQ ID NO:25 and the probe of sequence SEQ ID NO:35;
i) NPR3 gene by using the primers of sequences SEQ ID NO:26 and SEQ ID NO:27 and the probe of sequence SEQ ID NO:36;
and
b) comparing the level of methylation of the selected gene(s) to a reference value;
c) predicting the clinical outcome on the basis of the comparison of step b).

18. An array comprising at least one of the probes set forth in Table 4.

19. A kit comprising primers targeting at least one of the genes selected in the group consisting of: MROH6 gene, HOXB4 gene, OPLAH gene, CRCT1 gene, KCNQ4 gene, GP5 gene, NPR3 gene, chr7:129425301 gene and chr20:1784267 gene, preferably the primers set forth in Table 4.


SEQUENCE LISTING

<110> LES LABORATOIRES SERVIER
      UNIVERSITE DE PARIS
      CENTRE NATIONAL DE LA RECHERCHE SCIENTIFIQUE
      INSTITUT NATIONAL DE LA SANTE ET DE LA RECHERCHE MEDICALE
      ASSISTANCE PUBLIQUE HOPITAUX DE PARIS
      SORBONNE UNIVERSITE

<120> BIOMARKERS FOR DIAGNOSING AND MONITORING LUNG CANCER

<130> ctDNAlung

<160> 36

<170> PatentIn version 3.5

<210> 1
<211> 323
<212> DNA
<213> Homo sapiens

<400> 1
ccgtcacacc tgccactgag gtccttggga tgggtgagtc cctgacctgt aattgtcgga 60

ggggaggcac ggtgggagtg gtgagtgttg gatggcatag gggtgggatg gtgtcggggg 120

ctgctgacct cagcagcgtc cctgctgggg tcatgcaggc gcagcagcag cggcacgaga 180

ctctgcagca ccagcttccg cagggggccg cggagcccca gccggagccc gccccggccc 240

cggcgcacca gagtcccaag gagcccgacg gccgaggcgc ggattgagtc ccgtgtctgc 300

gtgggagggc gcagtcaggg cag 323

<210> 2
<211> 170
<212> DNA
<213> Homo sapiens

<400> 2
cgctccctcc cgccgagggg aggagactgg ggcctttgga aggtacagaa tgctgggcag 60

aggtgaggca gaacaggagg gctacaccgg gagccccaac ttcttaaggg atctagggtt 120

tgcgctctgc ctagcgcccc aacaggccct ccagtcgcaa cctgcgcgcg 170

<210> 3
<211> 714
<212> DNA

<213>  Homo sapiens

<400>  3
tcgtctgtaa attgggagct tacccaggtc ggaggactgt tggaattgga aatattcgaa      60

taaggaagtg tttttgcaag tgctttgtaa gcagcaaagc gcttcttcag gggtcaattt     120

ttttttagct ctgcagtcac cacccaaatt cggaagatcg ttgtgccttt cttggatgag     180

aatgcccggc tccagcccgg ggatgggacg gtcttttcct tgctaggagt ccgctgtgga     240

gggggcgatg cgctctgtcc aggacctagg agtccctttc tccctccacc ctcggctcac     300

agcgggcagc tcccccgacg cgtaccgcct ctttgccccc agcctgaggt gcgctcttcc     360

ccaggacgcc tcctctgtcc tctgcacgcc gccggccctg ctggggttgc tttctgccgc     420

ggcgtcttcg cgaatatccc ctcttgctgt ctgcctgggc gtccctccgg gttaccgcac     480

accgcctccg accctcccag ggcgcgggag gaggggcagg gcatccgcgt tcgtgtctgg     540

gccctggcgg ggagtcgcgt ggtccaaccc ctcgttttta agaggaggga tctgaagccc     600

agctaggctt ctaattccag acagagccct acggagccca gttctctgga ggctttcctg     660

gggctttttt cactaggcca ggttgggtgg gtctggagag aactcccgca accg           714

<210>  4
<211>  193
<212>  DNA
<213>  Homo sapiens

<400>  4
cggggcgccg cggtaggggc actggagcgc acgatgcacc ccgccaacga gtcctttctg      60

cagacggggt tcctgttttc atgcaaatgc ctttgttagc gcaccgggaa ccagggggac     120

ggaactgcag ctgacgcggg ctgcgcgccg cttttccgcc ttcgcttgat tcggcctcaa     180

cgacttaaac gcg                                                        193

<210>  5
<211>  228
<212>  DNA
<213>  Homo sapiens

<400>  5
cgccaacagg atgtgttctg tctccacacg cccggcggcg gtggctatgg ggacccggag      60

gaccccgccc caccgccggg gtcgcccccg caagcactgg cctttcccga gcacggcagc     120

gtctatgagt atcgccgggc ccaggaggcc gtgtgaggat cccgcaataa agatgcctta 180

agtctcccgg ttctggggac gcagctacgg cgcctagttc gtgctccg 228

<210> 6
<211> 195
<212> DNA
<213> Homo sapiens

<400> 6
cgcctgggac tggctctgtg ggctggccgt ccctctggct ggggacacgg ggaggccgag 60

cgtgtccgca aagacacgcg agctgtccac ctttctgggc tgcaggggtc tgcagaacct 120

cggaacctcg gaggggctct ctgtctgctt cagcgtctct ttctcaagtg actgaaagca 180

gaggctccct gctgc 195

<210> 7
<211> 114
<212> DNA
<213> Homo sapiens

<400> 7
ccgcacagat tcagcggacc ctccccctcc ctccccagtc ccctgccaca tccccagaag 60

tccccgcctc cgggtccgtg cgcggggtag gctggctgtg atctcgccgc cccc 114

<210> 8
<211> 139
<212> DNA
<213> Homo sapiens

<400> 8
cgcgaacgct cctgtgtgtt gaccacactc ccacggttgc tttttcagac atgctgaggg 60

ggactctact gtgcgcggtg ctcgggcttc tgcgcgccca gcccttcccc tgtccgccag 120

cttgcaagtg tgtcttccg 139

<210> 9
<211> 737
<212> DNA
<213> Homo sapiens

<400> 9
gcgacactca cttctcccag agtgttctgc aacgcagttt ctaatgcgcc cagagctaag 60

ctttgcggga gtgccttttg aagaaacgag cgctgaacct tctttccagc gacactcact 120

tctcccagag tgttctgcaa cgcagtttct aatgcgccca gagctaagct ttgcgggagt 180

gccttttgaa gaaacgagcg ctgaaccttc tttccacctg tcccccaagt tttctccatg 240

ggtctttggg tgttggaata aaacccaaac tggacaaaga gctcgaggct gaggatggtg 300

gctatggctt cggggagcgg ggggacgcgg acgtgtaatc gccagtgtgg cccattttac 360

ggtagggtag aatctgaggg aaggcggact gagatgccgg tatagcgccg atccctcctc 420

cctctagtca gggattagga gcactgcgat gagggaatcc cgtctctgct cccccttggc 480

gctcacgcgc ctggaatgtg agtggtgcaa tcccggcaaa gctccccgag accccagtgc 540

cacgcggatg agaccgaagg ggagcggagt tgagccaggc gccgggctag tccacaactt 600

ggcatggccg agcacctggg cgctggtcgt aggggacagc gcaacctgag gccgaaggcc 660

ccaggaacag gtgagagtgg acagagaagc catcgataat ggcgctgctg tcaccgcctt 720

gccatcttgg ccacctc 737

<210> 10
<211> 24
<212> DNA
<213> Artificial Sequence

<220>
<223> primer

<400> 10
agtagcggta cgagattttg tagt 24

<210> 11
<211> 23
<212> DNA
<213> Artificial Sequence

<220>
<223> primer

<400> 11
ctctaatacg ccgaaaccga aac 23

<210> 12
<211> 20

<212>  DNA
<213>  Artificial Sequence

<220>
<223>  primer

<400>  12
gggtttgcgt tttgtttagc                                                    20


<210>  13
<211>  20
<212>  DNA
<213>  Artificial Sequence

<220>
<223>  primer

<400>  13
ttacctcgct ctctccgaaa                                                    20


<210>  14
<211>  20
<212>  DNA
<213>  Artificial Sequence

<220>
<223>  primer

<400>  14
tttgtacgtc gtcggttttg                                                    20


<210>  15
<211>  19
<212>  DNA
<213>  Artificial Sequence

<220>
<223>  primer

<400>  15
acgcccaaac aaacaacaa                                                     19


<210>  16
<211>  20
<212>  DNA
<213>  Artificial Sequence

<220>

<223>  primer

<400>  16
gtatcgggaa ttagggggac                                                    20


<210>  17
<211>  21
<212>  DNA
<213>  Artificial Sequence

<220>
<223>  primer

<400>  17
cgttaaaacc gaatcaaacg a                                                  21


<210>  18
<211>  20
<212>  DNA
<213>  Artificial Sequence

<220>
<223>  primer

<400>  18
cggggtcgtt ttcgtaagta                                                    20


<210>  19
<211>  20
<212>  DNA
<213>  Artificial Sequence

<220>
<223>  primer

<400>  19
gacctcctaa acccgacgat                                                    20


<210>  20
<211>  23
<212>  DNA
<213>  Artificial Sequence

<220>
<223>  primer

<400>  20
gtcgagcgtg ttcgtaaaga tac                                                23

<210>  21
<211>  21
<212>  DNA
<213>  Artificial Sequence

<220>
<223>  primer

<400>  21
acaaacaaaa aacccctccg a                                                21


<210>  22
<211>  24
<212>  DNA
<213>  Artificial Sequence

<220>
<223>  primer

<400>  22
ttagaagttt tcgttttcgg gttc                                             24


<210>  23
<211>  21
<212>  DNA
<213>  Artificial Sequence

<220>
<223>  primer

<400>  23
aaaacgacga aatcacaacc a                                                21


<210>  24
<211>  18
<212>  DNA
<213>  Artificial Sequence

<220>
<223>  primer

<400>  24
cgcgaacgtt tttgtgtg                                                    18


<210>  25
<211>  18

<212>  DNA
<213>  Artificial Sequence

<220>
<223>  primer

<400>  25
gcgcaaaaac ccgaacac                                                    18


<210>  26
<211>  20
<212>  DNA
<213>  Artificial Sequence

<220>
<223>  primer

<400>  26
cgtttggaat gtgagtggtg                                                  20


<210>  27
<211>  20
<212>  DNA
<213>  Artificial Sequence

<220>
<223>  primer

<400>  27
ctaacccgac gcctaactca                                                  20


<210>  28
<211>  19
<212>  DNA
<213>  Artificial Sequence

<220>
<223>  probe

<400>  28
aactccgact aaaactccg                                                   19


<210>  29
<211>  20
<212>  DNA
<213>  Artificial Sequence

<220>

<223>  probe

<400>  29
cgcgcaaatt acgactaaaa                                                    20


<210>  30
<211>  15
<212>  DNA
<213>  Artificial Sequence

<220>
<223>  probe

<400>  30
acgccgcgac aaaaa                                                         15


<210>  31
<211>  18
<212>  DNA
<213>  Artificial Sequence

<220>
<223>  probe

<400>  31
acccgcgtca actacaat                                                      18


<210>  32
<211>  21
<212>  DNA
<213>  Artificial Sequence

<220>
<223>  probe

<400>  32
ctcataaacg ctaccgtact c                                                  21


<210>  33
<211>  20
<212>  DNA
<213>  Artificial Sequence

<220>
<223>  probe

<400>  33
tccgaaattc tacaaacccc                                                    20

<210>  34
<211>  15
<212>  DNA
<213>  Artificial Sequence

<220>
<223>  probe

<400>  34
acctaccccg cgcac                                                        15


<210>  35
<211>  18
<212>  DNA
<213>  Artificial Sequence

<220>
<223>  probe

<400>  35
cgcgcacaat aaaatccc                                                     18


<210>  36
<211>  20
<212>  DNA
<213>  Artificial Sequence

<220>
<223>  probe

<400>  36
ctcgaaaaac tttaccgaaa                                                   20